# EUROPEAN PATENT APPLICATION

(11) **EP 2 594 277 A1**
(43) Date of publication of application: **22.05.2013**
(21) Application number: 11803674.8
(22) Date of filing: 07.07.2011
(51) Int. Cl.: A61K 33/26, A61K 9/20, A61K 47/32, A61K 47/38, A61P 3/00, A61P 7/00, A61P 13/12

(54) **TABLET CONTAINING FERRIC CITRATE**

(30) Priority: 20.07.2010 US 399868 P; 07.07.2010 JP 2010155290
(71) Applicant: Japan Tobacco, Inc., Tokyo 105-8422 (JP)
(72) Inventor: FUKUSHIMA, Masayoshi, Takatsuki-shi, Osaka 569-1125 (JP); ORITA, Kazuhiro, Takatsuki-shi, Osaka 569-1125 (JP); YAMANE, Shogo, Takatsuki-shi, Osaka 569-1125 (JP)
(74) Representative: Vossius & Partner
(86) International application number: PCT/JP2011/065629
(87) International publication number: WO 2012/005340

(57) **Abstract**

The present invention provides a new preparation which is a tablet containing (1) ferric citrate, (2) a polyvinyl alcohol-polyethylene glycol graft copolymer, and (3) a polyvinyl alcohol-acrylic acid-methyl methacrylate copolymer.

## Description

### Technical Field

The present invention relates to a tablet containing ferric citrate as a medicinal active ingredient. Back ground Art

A capsule containing ferric citrate is known to be effective for the treatment of hyperphosphatemia.

### SUMMARY OF THE INVENTION

### Problems to be Solved by the Invention

The problem is to provide a new preparation. It has been clarified that production of a tablet containing ferric citrate in a high content as an active ingredient is associated with problems of moldability during tableting, crack, disintegration property and dissolution property of tablet, and the like.

### Means of Solving the Problems

The present inventors have conducted intensive studies and found that a tablet containing ferric citrate as a medicinal active ingredient and partially pregelatinized starch, or a tablet containing ferric citrate in a high content as the medicinal active ingredient, and a pharmaceutically acceptable carrier can solve the above-mentioned problem.
The present inventors have further conducted intensive studies and found that a tablet containing (1) ferric citrate, (2) a polyvinyl alcohol-polyethylene glycol graft copolymer and (3) a polyvinyl alcohol-acrylic acid-methyl methacrylate copolymer can solve the above-mentioned problem, which resulted in the completion of the present invention.

Accordingly, the present invention provides the following.
[1] A tablet comprising ferric citrate as a medicinal active ingredient, and partially pregelatinized starch.
[2] The tablet of the above-mentioned [1], further comprising low-substituted hydroxypropylcellulose and/or crystalline cellulose.
[3] The tablet of the above-mentioned [1] or [2], further comprising trehalose.
[4] The tablet of any one of the above-mentioned [1] - [3], further comprising a polyvinyl alcohol-polyethylene glycol graft copolymer.
[5] The tablet of any one of the above-mentioned [2] - [4], wherein the low-substituted hydroxypropylcellulose and/or crystalline cellulose are/is contained at a ratio of 5 parts by mass - 20 parts by mass in total relative to 100 parts by mass of ferric citrate equivalent to anhydride.
[6] The tablet of any one of the above-mentioned [3] - [5], wherein the trehalose is contained at a ratio of 2 parts by mass - 15 parts by mass relative to 100 parts by mass of ferric citrate equivalent to anhydride.
[7] The tablet of any one of the above-mentioned [4] - [6], wherein the polyvinyl alcohol-polyethylene glycol graft copolymer is contained at a ratio of 2 parts by mass - 15 parts by mass relative to 100 parts by mass of ferric citrate equivalent to anhydride.
[8] The tablet of any one of the above-mentioned [1] - [7], wherein the ferric citrate equivalent to anhydride is contained at a ratio of not less than 70 parts by mass relative to 100 parts by mass of a plain tablet free of water content of the ferric citrate.
[9] The tablet of any one of the above-mentioned [1] - [8], wherein the partially pregelatinized starch is contained at a ratio of 3 parts by mass - 20 parts by mass relative to 100 parts by mass of ferric citrate equivalent to anhydride.
[10] The tablet of any one of the above-mentioned [1] - [9], which shows a dissolution ratio of the ferric citrate in a 30-min dissolution time of not less than 75% in a dissolution test according to the Japanese Pharmacopoeia, 15th Edition, Dissolution Test, Paddle Method, using the Japanese Pharmacopoeia, 15th Edition Dissolution Test Method, 2nd fluid as a test solution, and at a rotation number of 50 rpm.
[11] Use of the tablet of any one of the above-mentioned [1] - [10] as a prophylactic or therapeutic agent of hyperphosphatemia.
[12] A tablet comprising ferric citrate as a medicinal active ingredient, and a pharmaceutically acceptable carrier, wherein the content of the medicinal active ingredient is high.
[13] The tablet of the above-mentioned [12], wherein one of the pharmaceutically acceptable carriers is partially pregelatinized starch.

[14] The tablet of the above-mentioned [12] or [13], wherein the ferric citrate equivalent to anhydride is contained at a ratio of not less than 70 parts by mass relative to 100 parts by mass of a plain tablet free of water content of the ferric citrate.
[15] The tablet of the above-mentioned [13] or [14], wherein partially pregelatinized starch is contained at a ratio of 3 parts by mass - 20 parts by mass relative to 100 parts by mass of ferric citrate equivalent to anhydride.
[16] A tablet comprising ferric citrate as a medicinal active ingredient, which shows a dissolution ratio of the ferric citrate in a 30-min dissolution time of not less than 75% in a dissolution test according to the Japanese Pharmacopoeia, 15th Edition, Dissolution Test, Paddle Method, using the Japanese Pharmacopoeia, 15th Edition Dissolution Test Method, 2nd fluid as a test solution, and at a rotation number of 50 rpm.
[17] The tablet of any one of the above-mentioned [12] - [15], which shows a dissolution ratio of the ferric citrate in a 30-min dissolution time of not less than 75% in a dissolution test according to the Japanese Pharmacopoeia, 15th Edition, Dissolution Test, Paddle Method, using the Japanese Pharmacopoeia, 15th Edition Dissolution Test Method, 2nd fluid as a test solution, and at a rotation number of 50 rpm.
[18] Use of the tablet of any one of the above-mentioned [12] - [17] as a prophylactic or therapeutic agent of hyperphosphatemia.
[19] A tablet comprising ferric citrate as a medicinal active ingredient, partially pregelatinized starch and low-substituted hydroxypropylcellulose.
[20] The tablet of the above-mentioned [19], further comprising a polyvinyl alcohol-polyethylene glycol graft copolymer.
[21] The tablet of the above-mentioned [19] or [20], wherein the ferric citrate equivalent to anhydride is contained at a ratio of not less than 70 parts by mass relative to 100 parts by mass of a plain tablet free of water content of the ferric citrate.
[22] The tablet of any one of the above-mentioned [19] - [21], wherein the partially pregelatinized starch is contained at a ratio of 3 parts by mass - 20 parts by mass relative to 100 parts by mass of the ferric citrate equivalent to anhydride.
[23] The tablet of any one of the above-mentioned [1] - [10], [12] - [17] and [19] - [22], which is coated.
[24] The method for the prophylaxis or treatment of hyperphosphatemia, comprising using the tablet of any one of the above-mentioned [1] - [10], [12] - [17] and [19] - [23].
[25] A method of producing a tablet, comprising a step of mixing or granulating ferric citrate as a medicinal active ingredient and partially pregelatinized starch.
[26] A production method comprising the step of the above-mentioned [25] and further comprising a step of adding a polyvinyl alcohol-polyethylene glycol graft copolymer and mixing or granulating the mixture.
[27] The production method of the above-mentioned [25] or [26], further comprising a step of adding and mixing low-substituted hydroxypropylcellulose.
[28] The production method of any one of the above-mentioned [25] - [27], further comprising a compression molding step.
[29] The production method of any one of the above-mentioned [25] - [28], further comprising a coating step.
[30] A tablet produced by the production method of any one of the above-mentioned [25] - [29].

[30-2] A tablet comprising (1) ferric citrate and further (2) a polyvinyl alcohol-polyethylene glycol graft copolymer and/or a polyvinyl alcohol-acrylic acid-methyl methacrylate copolymer.
[31] A tablet comprising (1) ferric citrate, (2) a polyvinyl alcohol-polyethylene glycol graft copolymer and (3) a polyvinyl alcohol-acrylic acid-methyl methacrylate copolymer.
[32] The tablet of the above-mentioned [31], further comprising one or more selected from the group consisting of low-substituted hydroxypropylcellulose, crystalline cellulose (microcrystalline cellulose) and carboxymethylcellulose.
[33] The tablet of the above-mentioned [31] or [32], further comprising a lubricant.
[34] The tablet of the above-mentioned [33], wherein the lubricant comprises calcium stearate and/or magnesium stearate.
[35] The tablet of any one of the above-mentioned [31] - [34], further comprising crospovidone.
[36] The tablet of any one of the above-mentioned [31] - [35], wherein the ferric citrate equivalent to anhydride is contained at a ratio of not less than 70 parts by mass relative to 100 parts by mass of a plain tablet free of water content of the ferric citrate.
[37] The tablet of any one of the above-mentioned [31] - [36], which is coated.
[38] Use of the tablet of any one of the above-mentioned [31] - [37] as a prophylactic or therapeutic agent of hyperphosphatemia.
[39] A tablet comprising ferric citrate as a medicinal active ingredient and a pharmaceutically acceptable carrier, wherein the content of the medicinal active ingredient is high.
[40] The tablet of the above-mentioned [39], wherein the pharmaceutically acceptable carrier is comprised of a polyvinyl alcohol-polyethylene glycol graft copolymer and a polyvinyl alcohol-acrylic acid-methyl methacrylate copolymer.
[41] The tablet of the above-mentioned [39] or [40], further comprising one or more selected from the group consisting of low-substituted hydroxypropylcellulose, crystalline cellulose and carboxymethylcellulose.
[42] The tablet of any one of the above-mentioned [39] - [41], further comprising a lubricant.
[43] The tablet of any one of the above-mentioned [39] - [42], further comprising crospovidone.
[44] The tablet of any one of the above-mentioned [39] - [43], wherein the ferric citrate equivalent to anhydride is contained at a ratio of not less than 70 parts by mass relative to 100 parts by mass of a plain tablet free of water content of the ferric citrate.
[45] The tablet of any one of the above-mentioned [39] - [44], which is coated.
[46] Use of the tablet of any one of the above-mentioned [39] - [45] as a prophylactic or therapeutic agent of hyperphosphatemia.
[47] The tablet of any one of the above-mentioned [30-2], [31] - [37] and [39] - [45], which shows a dissolution ratio of the ferric citrate in a 30-min dissolution time of not less than 75% in a dissolution test according to the Japanese Pharmacopoeia, 15th Edition, Dissolution Test, Paddle Method, using the Japanese Pharmacopoeia, 15th Edition Dissolution Test Method, 2nd fluid as a test solution, and at a rotation number of 50 rpm.
[48] A method of producing a tablet, comprising a step of mixing or granulating ferric citrate as a medicinal active ingredient, a polyvinyl alcohol-polyethylene glycol graft copolymer and a polyvinyl alcohol-acrylic acid-methyl methacrylate copolymer.
[49] The production method of the above-mentioned [48], further comprising a step of adding and mixing low-substituted hydroxypropylcellulose.
[50] The production method of the above-mentioned [48] or [49], further comprising a compression molding step.
[51] The production method of any one of the above-mentioned [48] - [50], further comprising a coating step.

In the present specification, the tablets of the above-mentioned [1] - [10], [12] - [17], [19] - [23] and [30] are also referred to as tablet (I).
In the present specification, the tablets of the above-mentioned [30-2], [31] - [37], [39] - [45] and [47] are also referred to as tablet (II).

### Effect of the Invention

The present invention can provide a new preparation containing an effective amount of ferric citrate.

### Brief Description of the Drawings

Fig. 1A shows the results of Experimental Example 1A regarding the tablets of Examples 1A and 2A.
Fig. 2A shows the results of Experimental Example 1A regarding the tablets of Examples 3A and 4A.
Fig. 3A shows the results of Experimental Example 2A regarding the tablets of Examples 5A and 6A.
Fig. 4A shows the results of Experimental Example 2A regarding the tablets of Examples 7A and 8A.
Fig. 5A shows the results of Experimental Example 3A.
Fig. 6A shows the results of Experimental Example 4A.
Fig. 7A shows the results of Experimental Example 8A.
Fig. 8A shows the results of Experimental Example 9A.
Fig. 9A shows the results of Experimental Example 10A.

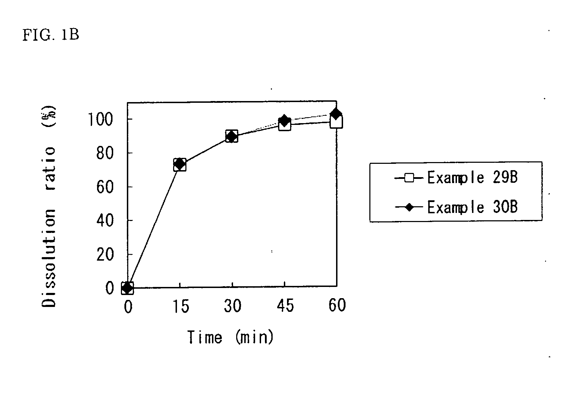

Fig. 1B shows the results of Experimental Example 13B.

### Description of Embodiments

The definitions used in the present invention are as follows.
In the present invention, the "ferric citrate" used as a medicinal active ingredient may be a water-containing material or anhydride. The form of the "ferric citrate" is not particularly limited.

The ferric citrate to be used in the present invention can be produced by a method known per se, for example, the method described in WO2004/07444 or a method analogous thereto.

To be specific, for example, ferric citrate can be produced by including the following steps (a) - (f). Each condition and the like of steps (a) - (f) may be according to those in the method described in WO2004/07444
(a) Acquiring ferric chloride hexahydrate,
(b) adding sodium hydroxide to ferric chloride hexahydrate at a rate and temperature effective for preparing a uniform polyiron oxo suspension,
(c) isolating a precipitate from the suspension,
(d) adding crystalline citric acid to the precipitate,
(e) forming a ferric-citric acid solution by heating citric acid and the precipitate, and
(f) adding an organic solvent to the ferric-citric acid solution to precipitate ferric citrate from the solution.
Each step can be changed as appropriate.

In tablets (I) and tablet (II) of the present invention, ferric citrate equivalent to anhydride is contained at a ratio of preferably 70 parts by mass - 90 parts by mass, more preferably 70 parts by mass - 85 parts by mass, particularly preferably 70 parts by mass - 80 parts by mass, relative to 100 parts by mass of a plain tablet free of water content of the ferric citrate.
In another embodiment of tablets (I) and tablet (II) of the present invention, ferric citrate equivalent to anhydride may be contained at a ratio of preferably not less than 70 parts by mass, more preferably not less than 80 parts by mass, particularly preferably not less than 85 parts by mass, relative to 100 parts by mass of a plain tablet free of water content of the ferric citrate.
Here, in the present specification, when the tablet is a coated tablet, the "plain tablet" means a plain tablet before coating, and the tablet per se when it is not coated.
In the present specification, moreover, by the "comprising the medicinal active ingredient in a high content" is meant that not less than 70 parts by mass of ferric citrate equivalent to anhydride is preferably contained relative to 100 parts by mass of a plain tablet free of water content of the ferric citrate. The present invention can provide a tablet comprising ferric citrate in a high content and, as a result, a tablet can be downsized and/or the number of tablets to be administered can be reduced, whereby administration compliance can be improved.
The "anhydride equivalent" is an amount corresponding to a solid of ferric citrate. Specifically, it refers to an amount of a solid content ferric citrate, which is obtained by measuring the water content of ferric citrate by the Karl Fischer's method, and removing the water content.
For example, in the case of Example 12A, the mass of ferric citrate used as a starting material is 584.1 mg per tablet, and the amount of the solid content of ferric citrate (that is, amount of ferric citrate equivalent to anhydride), which is obtained by subtracting the water content (84.1 mg) measured by the Karl Fischer's method from the mass, is 500.0 mg. The mass of the "plain tablet free of water content of the ferric citrate" in Example 12A is the mass 573.5 mg per tablet, which is obtained by subtracting the above-mentioned 84.1 mg from the total mass of the starting material of the plain tablet (657.6 mg). Thus, in the plain tablet of Example 12A, the "ferric citrate equivalent to anhydride" is contained at a ratio of 87.2 parts by mass (500.0 mg/573.5 mg x 100) relative to 100 parts by mass of the "plain tablet free of water content of the ferric citrate".

In tablet (I) of the present invention, the "partially pregelatinized starch" is obtained, for example, by heating starch together with water under normal pressure or pressurization to partially pregelatinize starch particles and drying them. Examples of the starch to be the starting material include corn starch, potato starch, rice starch and the like, with preference given to corn starch. As the "partially pregelatinized starch" to be used for tablet (I) of the present invention, the partially pregelatinized starch described in the Japanese Pharmaceutical Excipients 2003 (YAKUJI NIPPO LIMITED., published in 2003) is preferable.
The "partially pregelatinized starch" to be used for tablet (I) of the present invention can be produced by a known method or a commercially available product may also be used. Examples of the commercially available product include Starch 1500G (manufactured by Japan Colorcon), PCS (manufactured by Asahikasei Chemicals) and the like, with preference given to Starch 1500G (manufactured by Japan Colorcon).

In tablet (I) of the present invention, the mass ratio of ferric citrate and partially pregelatinized starch is, for example, partially pregelatinized starch being 3 parts by mass - 20 parts by mass relative to 100 parts by mass of ferric citrate equivalent to anhydride. Furthermore, partially pregelatinized starch may be, for example, not less than 3.5 parts by mass, not less than 5.9 parts by mass or not less than 8.2 parts by mass relative to 100 parts by mass of ferric citrate equivalent to anhydride. In addition, partially pregelatinized starch may be, for example, not more than 17.6 parts by mass relative to 100 parts by mass of ferric citrate equivalent to anhydride.

The tablet (I) of the present invention preferably contains low-substituted hydroxypropylcellulose and/or crystalline cellulose.
In tablet (I) of the present invention, the "low-substituted hydroxypropylcellulose" may be, but is not limited to, for example, low-substituted hydroxypropylcellulose defined in the Japanese Pharmacopoeia, 15th Edition.
The "low-substituted hydroxypropylcellulose" to be used for tablet (I) of the present invention can be produced according to a known method or a commercially available product may also be used. Examples of the commercially available product include L-HPC (grade: LH-11, LH-21, LH-22, LH-31, LH-32, LH-B1, manufactured by Shin-Etsu Chemical Co., Ltd.) and the like, with preference given to Lah-11 (manufactured by Shin-Etsu Chemical Co., Ltd.)

In tablet (I) of the present invention, the "crystalline cellulose" is a concept encompassing microcrystalline cellulose.
In tablet (I) of the present invention, the "crystalline cellulose" may be, but is not limited to, for example, crystalline cellulose defined in the Japanese Pharmacopoeia, 15th Edition.
The "crystalline cellulose" to be used for tablet (I) of the present invention can be produced by a known method or a commercially available product may also be used. Examples of the commercially available product include CEOLUS (grades: PH-101, PH-102, PH-301, PH-302, KG-802, KG-1000, manufactured by Asahikasei Chemicals), VIVAPUR (grades: 101, 102, 105, 301, 302, manufactured by JRS Pharma), Emcocel (grade: 50M, 90M, manufactured by JRS Pharma) and the like, with preference given to CEOLUS KG-1000 (manufactured by Asahikasei Chemicals).

When low-substituted hydroxypropylcellulose and/or crystalline cellulose are/is used for tablet (I) of the present invention, the mass ratio of ferric citrate and low-substituted hydroxypropylcellulose and/or crystalline cellulose is low-substituted hydroxypropylcellulose and/or crystalline cellulose in total of, for example, 5 parts by mass - 20 parts by mass relative to 100 parts by mass of ferric citrate equivalent to anhydride. Moreover, low-substituted hydroxypropylcellulose and/or crystalline cellulose in total may be, for example, not less than 8 parts by mass, or not less than 10 parts by mass, relative to 100 parts by mass of ferric citrate equivalent to anhydride. Or, low-substituted hydroxypropylcellulose and/or crystalline cellulose in total may be, for example, not less than 15 parts by mass relative to 100 parts by mass of ferric citrate equivalent to anhydride.

The tablet (I) of the present invention preferably contains trehalose.
The "trehalose" to be used for tablet (I) of the present invention can be produced by a known method or a commercially available product may also be used. Examples of the commercially available product include trehalose (grade: P, G, manufactured by Asahikasei Chemicals) and the like.

When trehalose is used for tablet (I) of the present invention, the mass ratio of ferric citrate and trehalose is, for example, 2 parts by mass - 15 parts by mass of trehalose relative to 100 parts by mass of ferric citrate equivalent to anhydride. Furthermore, trehalose may be, for example, not less than 5 parts by mass relative to 100 parts by mass of ferric citrate equivalent to anhydride. Trehalose may be, for example, not more than 12 parts by mass relative to 100 parts by mass of ferric citrate equivalent to anhydride.

Tablet (I) of the present invention preferably contains a polyvinyl alcohol-polyethylene glycol graft copolymer.
In tablet (I) of the present invention, examples of the "polyvinyl alcohol-polyethylene glycol graft copolymer" include, but is not limited to, a graft copolymer having an average molecular weight of about 45000, which is constituted at a ratio of polyethylene glycol (1 mol) to polyvinyl alcohol (3 mol).
The "polyvinyl alcohol-polyethylene glycol graft copolymer" to be used for tablet (I) of the present invention can be produced by a known method or a commercially available product may also be used. Examples of the commercially available product include Kollicoat IR (manufactured by BASF) , with preference given to Kollicoat IR (manufactured by BASE).

When a polyvinyl alcohol-polyethylene glycol graft copolymer is used for tablet (I) of the present invention, the mass ratio of ferric citrate and the polyvinyl alcohol-polyethylene glycol graft copolymer is, for example, 2 parts by mass - 15 parts by mass of the polyvinyl alcohol-polyethylene glycol graft copolymer relative to 100 parts by mass of ferric citrate equivalent to anhydride. Moreover, it may be, for example, not less than 5 parts by mass relative to 100 parts by mass of ferric citrate equivalent to anhydride. Furthermore, it may be, for example, not more than 12 parts by mass relative to 100 parts by mass of ferric citrate equivalent to anhydride.

The tablet (I) of the present invention can further use a pharmaceutically acceptable carrier as necessary, besides the above-mentioned components. The amount of the pharmaceutically acceptable carrier to be used besides the above-mentioned components is not particularly limited.
In tablet (I) of the present invention, examples of the "pharmaceutically acceptable carrier" include carriers conventionally used in the field of pharmaceutical preparation, for example, additives such as excipient, disintegrant, binder, fluidizer, lubricant, preservative, antioxidant, colorant, sweetening agent and the like.
These additives can be used together with partially pregelatinized starch as necessary.

Examples of the "excipient" to be used for tablet (I) of the present invention include lactose, sucrose, D-mannitol, D-sorbitol, corn starch, dextrin, carboxymethylcellulose, calcium carboxymethylcellulose, sodium carboxymethyl starch, gum arabic and the like.
Examples of the "disintegrant" to be used for tablet (I) of the present invention include carboxymethylcellulose, calcium carboxymethylcellulose, sodium carboxymethylcellulose, sodium carboxymethyl starch, croscarmellose sodium, crospovidone and the like.
Examples of the "blinder" to be used for tablet (I) of the present invention include hydroxypropylcellulose, hypromellose, polyvinylpyrrolidone, sucrose, dextrin, starch, pregelatinized starch, gelatin, sodium carboxymethylcellulose, gum arabic and the like.
Examples of the "fluidizer," to be used for tablet (I) of the present invention include light anhydrous silicic acid, magnesium stearate and the like.
Examples of the "lubricant" to be used for tablet (I) of the present invention include magnesium stearate, calcium stearate, sodium stearyl fumarate, stearic acid, talc and the like.
Examples of the "preservative" to be used for tablet (I) of the present invention include ethyl parahydroxybenzoate, chlorobutanol, benzyl alcohol, sodium dehydroacetate, sorbic acid and the like.
Examples of the "antioxidant" to be used for tablet (I) of the present invention include sodium sulfite, ascorbic acid and the like.
Examples of the "colorant" to be used for tablet (I) of the present invention include food colors (e.g., Food Color Red No. 2 or No. 3, Food Color Yellow No. 4 or No. 5 etc.), β-carotene and the like.
Examples of the "sweetening agent" to be used for tablet (I) of the present invention include saccharin sodium, dipotassium glycyrrhizinate, aspartame and the like.

Examples of the "polyvinyl alcohol-polyethylene glycol graft copolymer" to be used for tablet (II) of the present invention include, but are not limited to, a graft copolymer having an average molecular weight of about 45000, which is constituted at a ratio of 1 mol of polyethylene glycol to 3 mol of polyvinyl alcohol as mentioned above.
The "polyvinyl alcohol-polyethylene glycol graft copolymer" to be used for tablet (II) of the present invention can be produced by a known method, or a commercially available product may also be used. Examples of the commercially available product include Kollicoat IR (manufactured by BASF), with preference given to Kollicoat IR (manufactured by BASF).
In tablet (II) of the present invention, the mass ratio of ferric citrate and polyvinyl alcohol-polyethylene glycol graft copolymer is, for example, 0.01 parts by mass - 15 parts by mass of the polyvinyl alcohol-polyethylene glycol graft copolymer relative to 100 parts by mass of ferric citrate equivalent to anhydride. Furthermore, it may be, for example, not less than 0.1 part by mass, or not less than 0.5 parts by mass, relative to 100 parts by mass of ferric citrate equivalent to anhydride. Moreover, it may be, for example, not more than 12 parts by mass, or not more than 10 parts by mass, relative to 100 parts by mass of ferric citrate equivalent to anhydride.
In tablet (II) of the present invention, the mass ratio of ferric citrate and the polyvinyl alcohol-polyethylene glycol graft copolymer is preferably 1 part by mass - 12 parts by mass of polyvinyl alcohol-polyethylene glycol graft copolymer relative to 100 parts by mass of ferric citrate equivalent to anhydride, more preferably, 1 parts by mass - 10 parts by mass of polyvinyl alcohol-polyethylene glycol graft copolymer relative to 100 parts by mass of ferric citrate equivalent to anhydride, particularly preferably, 3 parts by mass - 6 parts by mass, more particularly preferably, 3 parts by mass - 5 parts by mass, of polyvinyl alcohol-polyethylene glycol graft copolymer relative to 100 parts by mass of ferric citrate equivalent to anhydride.

In tablet (II) of the present invention, the "polyvinyl alcohol-acrylic acid-methyl methacrylate copolymer" is a copolymer comprised of polyvinyl alcohol, acrylic acid, and methyl methacrylate. Each polymerization ratio is not particularly limited as long as the polyvinyl alcohol-acrylic acid-methyl methacrylate copolymer shows a binding force. For example, 75% by mass - 80% by mass of polyvinyl alcohol, 2.0% by mass - 8.0% by mass of acrylic acid, and 17% by mass - 21% by mass of methyl methacrylate are suitable. Preferably, polyvinyl alcohol is 75% by mass - 80% by mass, acrylic acid is 2.5% by mass - 7.5% by mass, and methyl methacrylate is 17.5% by mass - 20% by mass.
Polyvinyl alcohol which is one of the constituent components of the polyvinyl alcohol-acrylic acid-methyl methacrylate copolymer to be used for tablet (II) of the present invention shows, for example, a degree of polymerization of 400 - 600, preferably 450 - 550, and a saponification value of 85 mol% - 90 mol%, preferably 86 mol% - 89 mol%.
The polyvinyl alcohol-acrylic acid-methyl methacrylate copolymer includes commercially available POVACOAT (manufactured by Daido Chemical Corporation) comprised of polyvinyl alcohol having a degree of polymerization of 500 and a saponification value of 86.5 mol% - 89.0 mol%, acrylic acid and methyl methacrylate copolymerized at a mass ratio of 80.0% by mass, 2.5% by mass and 17.5% by mass, respectively. For example, POVACOAT Type:F can be mentioned.

In tablet (II) of the present invention, the mass ratio of ferric citrate and polyvinyl alcohol-acrylic acid-methyl methacrylate copolymer is polyvinyl alcohol-acrylic acid-methyl methacrylate copolymer of, for example, 0.01 part by mass - 10 parts by mass relative to 100 parts by mass of ferric citrate equivalent to anhydride. Moreover, the polyvinyl alcohol-acrylic acid-methyl methacrylate copolymer may also be, for example, not less than 0.1 part by mass or not less than 0.5 parts by mass, relative to 100 parts by mass of ferric citrate equivalent to anhydride. In addition, the polyvinyl alcohol-acrylic acid-methyl methacrylate copolymer may also be, for example, not more than 8 parts by mass or not more than 6 parts by mass relative to 100 parts by mass of ferric citrate equivalent to anhydride.
In tablet (II) of the present invention, the mass ratio of the ferric citrate and polyvinyl alcohol-acrylic acid-methyl methacrylate copolymer is preferably 0.1 part by mass - 5 parts by mass of the polyvinyl alcohol-acrylic acid-methyl methacrylate copolymer relative to 100 parts by mass of ferric citrate equivalent to anhydride, more preferably, 0.1 part by mass - 3 parts by mass of the polyvinyl alcohol-acrylic acid-methyl methacrylate copolymer relative to 100 parts by mass of ferric citrate equivalent to anhydride, particularly preferably, 0.5 part by mass - 2 parts by mass, and further particularly preferably 1 part by mass - 2 parts by mass, of the polyvinyl alcohol-acrylic acid-methyl methacrylate copolymer relative to 100 parts by mass of ferric citrate equivalent to anhydride.

In tablet (II) of the present invention, when the "polyvinyl alcohol-polyethylene glycol graft copolymer and polyvinyl alcohol-acrylic acid-methyl methacrylate copolymer" are used, the mass ratio of ferric citrate and the "polyvinyl alcohol-polyethylene glycol graft copolymer and polyvinyl alcohol-acrylic acid-methyl methacrylate copolymer" is, for example, 3 parts by mass - 15 parts by mass of the "polyvinyl alcohol-polyethylene glycol graft copolymer and polyvinyl alcohol-acrylic acid-methyl methacrylate copolymer" relative to 100 parts by mass of ferric citrate equivalent to anhydride.
Moreover, the "polyvinyl alcohol-polyethylene glycol graft copolymer and polyvinyl alcohol-acrylic acid-methyl methacrylate copolymer" may be used in, for example, not less than 5 parts by mass relative to 100 parts by mass of ferric citrate equivalent to anhydride.
Furthermore, the "polyvinyl alcohol-polyethylene glycol graft copolymer and polyvinyl alcohol-acrylic acid-methyl methacrylate copolymer" may be used in, for example, not more than 12 parts by mass relative to 100 parts by mass of ferric citrate equivalent to anhydride.
In tablet (II) of the present invention, when the "polyvinyl alcohol-polyethylene glycol graft copolymer and polyvinyl alcohol-acrylic acid-methyl methacrylate copolymer" is used, the mass ratio of ferric citrate and the "polyvinyl alcohol-polyethylene glycol graft copolymer and polyvinyl alcohol-acrylic acid-methyl methacrylate copolymer" is preferably 3 parts by mass - 10 parts by mass of the "polyvinyl alcohol-polyethylene glycol graft copolymer and polyvinyl alcohol-acrylic acid-methyl methacrylate copolymer" relative to 100 parts by mass of ferric citrate equivalent to anhydride, more preferably, 4 parts by mass - 7 parts by mass of the "polyvinyl alcohol-polyethylene glycol graft copolymer and polyvinyl alcohol-acrylic acid-methyl methacrylate copolymer" relative to 100 parts by mass of ferric citrate equivalent to anhydride, and particularly preferably, 5 parts by mass - 7 parts by mass of the "polyvinyl alcohol-polyethylene glycol graft copolymer and polyvinyl alcohol-acrylic acid-methyl methacrylate copolymer" relative to 100 parts by mass of ferric citrate equivalent to anhydride.

The tablet (II) of the present invention preferably contains one or more selected from the group consisting of low-substituted hydroxypropylcellulose, crystalline cellulose and carboxymethylcellulose.

Examples of the "low-substituted hydroxypropylcellulose" to be used for tablet (II) of the present invention include, but are not limited to, low-substituted hydroxypropylcellulose defined in the Japanese Pharmacopoeia, 15th Edition, as mentioned above.
The "low-substituted hydroxypropylcellulose" to be used for tablet (II) of the present invention can be produced by a known method, or a commercially available product may also be used. Examples of the commercially available product include L-HPC (grade: LH-11, LH-21, LH-22, LH-31, LH-32, LH-B1, manufactured by Shin-Etsu Chemical Co., Ltd.) and the like, with preference given to LH-11 (manufactured by Shin-Etsu Chemical Co., Ltd.).
In tablet (II) of the present invention, when low-substituted hydroxypropylcellulose is used, the mass ratio of ferric citrate and low-substituted hydroxypropylcellulose is, for example, 5 parts by mass - 20 parts by mass of low-substituted hydroxypropylcellulose relative to 100 parts by mass of ferric citrate equivalent to anhydride. Furthermore, low-substituted hydroxypropylcellulose may be used in, for example, not less than 8 parts by mass, or not less than 10 parts by mass, relative to 100 parts by mass of ferric citrate equivalent to anhydride. Moreover, low-substituted hydroxypropylcellulose may be used in, for example, not more than 15 parts by mass relative to 100 parts by mass of ferric citrate equivalent to anhydride.
In tablet (II) of the present invention, when low-substituted hydroxypropylcellulose is used, the mass ratio of ferric citrate and low-substituted hydroxypropylcellulose is preferably 6 parts by mass - 16 parts by mass of low-substituted hydroxypropylcellulose relative to 100 parts by mass of ferric citrate equivalent to anhydride, more preferably 8 parts by mass - 16 parts by mass of low-substituted hydroxypropylcellulose relative to 100 parts by mass of ferric citrate equivalent to anhydride, particularly preferably 10 parts by mass - 14 parts by mass of low-substituted hydroxypropylcellulose relative to 100 parts by mass of ferric citrate equivalent to anhydride.

In tablet (II) of the present invention, the "crystalline cellulose" is a concept encompassing microcrystalline cellulose.
Examples of the "crystalline cellulose" to be used for tablet (II) of the present invention include, but are not limited to, crystalline cellulose defined in the Japanese Pharmacopoeia, 15th Edition.
The "crystalline cellulose" to be used for tablet (II) of the present invention can be produced by a known method, or a commercially available product may also be used. Examples of the commercially available product include CEOLUS (grade: PH-101, PH-102, PH-301, PH-302, KG-802, KG-1000, manufactured by Asahikasei Chemicals), VIVAPUR (grade: 101, 102, 105, 301, 302, manufactured by JRS Pharma), Emcocel (grade: 50M, 90M, manufactured by JRS Pharma) and the like.

Examples of the "carboxymethylcellulose" to be used for tablet (II) of the present invention include, but are not limited to, carboxymethylcellulose defined in the Japanese Pharmacopoeia, 15th Edition.
Carboxymethylcellulose to be used for tablet (II) of the present invention can be produced by a known method, or a commercially available product may also be used. Examples of the commercially available product include NS-300 (manufactured by Nichirin Chemical Corporation) and the like, with preference given to NS-300 (manufactured by Nichirin Chemical Corpration).

In tablet (II) of the present invention, when one or more selected from the group consisting of low-substituted hydroxypropylcellulose, crystalline cellulose and carboxymethylcellulose is used, the mass ratio of ferric citrate and one or more selected from the group consisting of low-substituted hydroxypropylcellulose, crystalline cellulose and carboxymethylcellulose is, for example, 5 parts by mass - 42 parts by mass of one or more selected from the group consisting of low-substituted hydroxypropylcellulose, crystalline cellulose and carboxymethylcellulose relative to 100 parts by mass of ferric citrate equivalent to anhydride. Furthermore, one or more selected from the group consisting of low-substituted hydroxypropylcellulose, crystalline cellulose and carboxymethylcellulose may be used in, for example, not less than 8 parts by mass relative to 100 parts by mass of ferric citrate equivalent to anhydride. Moreover, one or more selected from the group consisting of low-substituted hydroxypropylcellulose, crystalline cellulose and carboxymethylcellulose may be used in, for example, not more than 36 parts by mass relative to 100 parts by mass of ferric citrate equivalent to anhydride.
In tablet (II) of the present invention, when one or more selected from the group consisting of low-substituted hydroxypropylcellulose, crystalline cellulose and carboxymethylcellulose is used, the mass ratio of ferric citrate and one or more selected from the group consisting of low-substituted hydroxypropylcellulose, crystalline cellulose and carboxymethylcellulose is preferably 10 parts by mass - 35 parts by mass of one or more selected from the group consisting of low-substituted hydroxypropylcellulose, crystalline cellulose and carboxymethylcellulose relative to 100 parts by mass of ferric citrate equivalent to anhydride. More preferably, one or more selected from the group consisting of low-substituted hydroxypropylcellulose, crystalline cellulose and carboxymethylcellulose is used in 20 parts by mass - 35 parts by mass relative to 100 parts by mass of ferric citrate equivalent to anhydride. Particularly preferably, one or more selected from the group consisting of low-substituted hydroxypropylcellulose, crystalline cellulose and carboxymethylcellulose is used in 25 parts by mass - 32 parts by mass relative to 100 parts by mass of ferric citrate equivalent to anhydride.

In tablet (II) of the present invention, "crospovidone" is a crosslinked polymer substance of 1-vinyl-2-pyrrolidone. Examples thereof include, but are not limited to, crospovidone defined in the Japanese Pharmaceutical Excipients 2003 (Yakuji Nippou Corporation, published in 2003).
Crospovidone to be used for tablet (II) of the present invention can be produced by a known method, or a commercially available product may also be used. Examples of the commercially available product include Kollidon (grade: CL, CL-F, CL-SF, CL-M, manufactured by BASF), Polyplasdone (grade: XL, XL-10, INF-10, manufactured by ISP) and the like, with preference given to Kollidon CL-F (manufactured by BASF) .
In tablet (II) of the present invention, when crospovidone is used, the mass ratio of ferric citrate and crospovidone is, for example, 0.01 part by mass - 10 parts by mass of crospovidone relative to 100 parts by mass of ferric citrate equivalent to anhydride. Further, crospovidone may be, for example, in not less than 0.1 part by mass, or not less than 0.5 part by mass, relative to 100 parts by mass of ferric citrate equivalent to anhydride. In addition, crospovidone may be, for example, in not more than 8 parts by mass relative to 100 parts by mass of ferric citrate equivalent to anhydride.
In tablet (II) of the present invention, when crospovidone is used, the mass ratio of ferric citrate and crospovidone is preferably 0.1 part by mass - 6 parts by mass of crospovidone relative to 100 parts by mass of ferric citrate equivalent to anhydride, more preferably, 0.5 part by mass - 4 parts by mass of crospovidone relative to 100 parts by mass of ferric citrate equivalent to anhydride.

The tablet (II) of the present invention can further use a pharmaceutically acceptable carrier as necessary, besides the above-mentioned components. The amount of the pharmaceutically acceptable carrier to be used besides the above-mentioned components is not particularly limited.
Examples of the "pharmaceutically acceptable carrier" to be used for tablet (II) of the present invention include carriers conventionally used in the field of pharmaceutical preparation, for example, additives such as excipient, disintegrant, binder, fluidizer, lubricant, preservative, antioxidant, colorant, sweetening agent and the like.
These additives can be used together with a polyvinyl alcohol-polyethylene glycol graft copolymer and a polyvinyl alcohol-acrylic acid-methyl methacrylate copolymer as necessary.

Examples of the "excipient" to be used for tablet (II) of the present invention include lactose, sucrose, D-mannitol, D-sorbitol, corn starch, dextrin, trehalose, calcium carboxymethylcellulose, sodium carboxymethyl starch, gum arabic and the like.
Examples of the "disintegrant" to be used for tablet (II) of the present invention include calcium carboxymethylcellulose, sodium carboxymethylcellulose, sodium carboxymethyl starch, croscarmellose sodium and the like.
Examples of the "binder" to be used for tablet (II) of the present invention include hydroxypropylcellulose, hypromellose, polyvinylpyrrolidone, sucrose, dextrin, starch, pregelatinized starch, partially pregelatinized starch, gelatin, sodium carboxymethylcellulose, gum arabic and the like.
Examples of the "fluidizer" to be used for tablet (II) of the present invention include light anhydrous silicic acid, magnesium stearate and the like.
Examples of the "lubricant" to be used for tablet (II) of the present invention include magnesium stearate, calcium stearate, sodium stearyl fumarate, stearic acid, talc and the like.
Examples of the "preservative" to be used for tablet (II) of the present invention include ethyl parahydroxybenzoate, chlorobutanol, benzyl alcohol, sodium dehydroacetate, sorbic acid and the like.
Examples of the "antioxidant" to be used for tablet (II) of the present invention include sodium sulfite, ascorbic acid and the like.
Examples of the "colorant" to be used for tablet (II) of the present invention include food colors (e.g., Food Color Red No. 2 or No. 3, Food Color Yellow No. 4 or No. 5 etc.), β-carotene and the like.
Examples of the "sweetening agent" to be used for tablet (II) of the present invention include saccharin sodium, dipotassium glycyrrhizinate, aspartame and the like.

The tablet (II) of the present invention preferably contains a lubricant. As the lubricant to be used for tablet (II) of the present invention, magnesium stearate or calcium stearate is preferable.
Magnesium stearate to be used for tablet (II) of the present invention can be produced by a known method, or a commercially available product may also be used. Examples of the commercially available product include magnesium stearate (manufactured by Taihei Chemical Industrial Co., Ltd., The Japanese Pharmacopoeia, plant-derived), magnesium stearate (manufactured by Nitto Kasei Kogyo), magnesium stearate (manufactured by Mallinckrodt) and the like.
Calcium stearate to be used for tablet (II) of the present invention can be produced by a known method, or a commercially available product may also be used. Examples of the commercially available product include calcium stearate (manufactured by Taihei Chemical Industrial Co., Ltd., The Japanese Pharmacopoeia, plant-derived), calcium stearate (manufactured by Nitto Kasei Kogyo), calcium stearate (manufactured by Mallinckrodt).
In tablet (II) of the present invention, when a lubricant is used, the mass ratio of ferric citrate and a lubricant is, for example, 0.01 part by mass - 6 parts by mass relative to 100 parts by mass of ferric citrate equivalent to anhydride. Furthermore, the lubricant may be, for example, in not less than 0.1 part by mass, or not less than 0.5 part by mass, relative to 100 parts by mass of ferric citrate equivalent to anhydride. Moreover, the lubricant may be, for example, in not more than 3 parts by mass relative to 100 parts by mass of ferric citrate equivalent to anhydride.

The tablet of the present invention may be coated with a coating agent as necessary.
As the "coating agent", coating agents conventionally used in the field of pharmaceutical preparations can be used. Examples thereof include hypromellose, hydroxypropylcellulose, polyvinyl alcohol and the like. The coating agent may be mixed with other additives such as plasticizers (e.g., macrogols, triacetine and the like), light shielding agents (e.g., titanium oxide and the like), attachment inhibitors (e.g., talc and the like) and the like as necessary and used for coating. Where necessary, a premixture coating agent such as opadry II (manufactured by Colorcon) containing a combination of other additives such as plasticizers (e.g., macrogols, triacetine and the like), light shielding agents (e.g., titanium oxide and the like) and the like may be used.

The "Japanese Pharmacopoeia, 15th Edition, Dissolution Test, 1st fluid" is obtained by dissolving sodium chloride (2.0 g) in hydrochloric acid (7.0 mL) and water to a total amount of 1000 mL.
The "Japanese Pharmacopoeia, 15th Edition, Dissolution Test, 2nd fluid" is obtained by adding water (1 volume) to 1 volume of a phosphate buffer (pH 6.8, potassium dihydrogen phosphate (3.40 g) and anhydrous disodium hydrogen phosphate (3.55 g) dissolved in water, to 1000 mL).
The "Japanese Pharmacopoeia, 15th Edition, Disintegration Test, 1st fluid" is the same as the "Japanese Pharmacopoeia, 15th Edition, Dissolution Test, 1st fluid".
The tablets (I) and (II) of the present invention preferably show a dissolution ratio of the ferric citrate in a 30-min dissolution time of not less than 70%, more preferably not less than 75%, in a dissolution test according to the Japanese Pharmacopoeia, 15th Edition, Dissolution Test, Paddle Method, using the Japanese Pharmacopoeia, 15th Edition Dissolution Test Method, 2nd fluid as a test solution, and at a rotation number of 50 rpm.
In a disintegration test according to the Japanese Pharmacopoeia, 15th Edition, Disintegration Test method, using the Japanese Pharmacopoeia, 15th Edition, Disintegration Test, 1st fluid as a test solution, the disintegration time of tablets (I) and (II) of the present invention is preferably not more than 10 min, more preferably not more than 5 min.

The tablet of the present invention can be used, for example, for the prophylaxis or treatment of hyperphosphatemia and the like.

While the primary subject of administration of the tablet of the present invention is human, it may be a mammal other than human (e.g., mouse, rat, hamster, guinea pig, rabbit, cat, dog, swine, bovine, horse, sheep, monkey etc.). The dose varies depending on the subject of administration, disease, symptom, administration route and the like.

The shape of the tablet of the present invention is not particularly limited and, for example, round-shaped tablet, irregularly-shaped tablet (e.g., capsule-shaped tablet (oval-shaped tablet)) and the like can be mentioned.

While the production method of the tablet of the present invention is explained below, though it is not limited thereto.
The production method of tablet (I) of the present invention includes a step of granulating or mixing ferric citrate as a medicinal active ingredient together with partially pregelatinized starch. Thereafter, the tablet of the present invention can be produced by compression molding by a method known per se.
Specifically, the following method is given as an example.

### 1. granulation or mixing step

Ferric citrate is granulated or mixed together with partially pregelatinized starch.
The granulation or mixing method is not particularly limited, and a method known per se in the pharmaceutical field can be used. For granulation, for example, fluid bed granulation and the like can be mentioned. Specifically, for example, using a fluid bed granulator and the like, partially pregelatinized starch is suspended in purified water and granulation can be performed while spraying the suspension on ferric citrate. After granulation, granules are dried to give dried granules. The obtained dried granules may be sieved where necessary. In addition, necessary additives may be added in any stage of the step and granulation or mixing may be performed.

### 2. compression molding step

To the obtained granulated product (e.g., granules) or a mixture is added a lubricant, and the mixture is compression molded to give the tablet of the present invention.
The method of compression molding the tablet is not particularly limited and, for example, a screw drive universal testing machine, a rotary tableting machine and the like are used. The compression molding force is not particularly limited as long as sufficient intensity can be applied to the tablet, and a tensile intensity of 1 N/mm² or more is preferable, a tensile intensity of 2 N/mm² or more is more preferable. Specifically, for a capsule-shaped tablet with major axis 14.8 mm and minor axis 6.8 mm, a tablet hardness of not less than 60N is preferable, and not less than 90N is more preferable.
The method of adding a lubricant may be an internal lubrication method or external lubrication method. In addition, a necessary additive may be added in any stage of this step. Moreover, coating with a coating agent may be applied after tableting.

When tablet (I) of the present invention contains one or more kinds selected from the group consisting of low-substituted hydroxypropylcellulose, crystalline cellulose, trehalose, and a polyvinyl alcohol-polyethylene glycol graft copolymer, the component may be added in any stage by and method. A preferable addition method is, for example, a method including addition to granules obtained by granulating ferric citrate together with partially pregelatinized starch.
For example, when tablet (I) of the present invention contains a polyvinyl alcohol-polyethylene glycol graft copolymer, the component may be added in any stage by and method. A preferable addition method is, for example, a method including granulating ferric citrate together with partially pregelatinized starch, further adding, to the obtained granules, a polyvinyl alcohol-polyethylene glycol graft copolymer and granulating the mixture.

The production method of tablet (II) of the present invention includes a step of granulating or mixing ferric citrate as a medicinal active ingredient together with polyvinyl alcohol-polyethylene glycol graft copolymer and polyvinyl alcohol-acrylic acid-methyl methacrylate copolymer. Thereafter, the tablet of the present invention can be produced by compression molding by a method known per se.
Specifically, the following method is given as an example.

### 1. granulation or mixing step

Ferric citrate is granulated or mixed together with polyvinyl alcohol-polyethylene glycol graft copolymer and polyvinyl alcohol-acrylic acid-methyl methacrylate copolymer.
The granulation or mixing method is not particularly limited, and a method known per se in the pharmaceutical field can be used. For granulation, for example, fluid bed granulation and the like can be mentioned. Specifically, for example, using a fluid bed granulator and the like, a polyvinyl alcohol-polyethylene glycol graft copolymer and a polyvinyl alcohol-acrylic acid-methyl methacrylate copolymer are dissolved in purified water and granulation can be performed while spraying the solution on ferric citrate. After granulation, granules are dried to give dried granules. The obtained dried granules may be sieved where necessary. In addition, necessary additives may be added in any stage of the step and granulation or mixing may be performed.

### 2. compression molding step

To the obtained granulated product (e.g., granules) or a mixture is added a lubricant, and the mixture is compression molded to give the tablet of the present invention.
The method of compression molding the tablet is not particularly limited and, for example, a screw drive universal testing machine, a rotary tableting machine and the like are used. The compression molding force is not particularly limited as long as sufficient intensity can be applied to the tablet, and a tensile intensity of 1 N/mm² or more is preferable, a tensile intensity of 1.5 N/mm² or more is more preferable.
The method of adding a lubricant may be an internal lubrication method or external lubrication method. In addition, a necessary additive may be added in any stage of this step. Moreover, coating with a coating agent may be applied after tableting.

When tablet (II) of the present invention contains crystalline cellulose, the component may be added in any stage by and method. A preferable addition method of crystalline cellulose is, for example, a method including granulating ferric citrate together with a polyvinyl alcohol-polyethylene glycol graft copolymer and a polyvinyl alcohol-acrylic acid-methyl methacrylate copolymer and adding to the obtained granules. In addition, crystalline cellulose may be added to ferric citrate, and granulated together with a polyvinyl alcohol-polyethylene glycol graft copolymer and a polyvinyl alcohol-acrylic acid-methyl methacrylate copolymer.
For example, when tablet (II) of the present invention contains low-substituted hydroxypropylcellulose and/or carboxymethylcellulose, the component may be added in any stage by and method. A preferable addition method is, for example, a method including granulating ferric citrate together with a polyvinyl alcohol-polyethylene glycol graft copolymer and a polyvinyl alcohol-acrylic acid-methyl methacrylate copolymer, and adding to the obtained granules.

The production method of the tablet of the present invention is explained more specifically by the following Examples, which are not to be construed as limiting the present invention. For production, the order of mixing and mixing conditions of the additives may be changed as appropriate. When the content of ferric citrate per tablet is changed, the mass of each additive can be changed according to the mass of ferric citrate.
In the Examples using magnesium stearate as an external lubricant, the content of magnesium stearate was considered to be zero.

### Examples

### Reference Example 1-1

Partially pregelatinized starch (Starch 1500G, manufactured by Japan Colorcon) was dispersed in purified water by using a propeller mixer, and uniformly dispersed using a handy homomixer. Purified water was added to prepare a binding solution with a concentration of 10% by mass.

### Reference Example 1-2

Hydroxypropylcellulose (HPC L, manufactured by Nippon Soda Co., Ltd.) was dissolved in purified water by using a propeller mixer to prepare a binding solution with a concentration of 5% by mass.

### Reference Example 1-3

Pregelatinized starch (Amycol C, manufactured by NIPPON STARCH CHEMICAL CO., LTD.) was dispersed in purified water by using a propeller mixer, and uniformly dispersed using a handy homomixer. Purified water was added to prepare a binding solution with a concentration of 5% by mass.

### Reference Example 1-4

Partially pregelatinized starch (Starch 1500G, manufactured by Japan Colorcon) was dispersed in purified water by using a propeller mixer, and sieved with a stainless sieve (aperture 250 µm). Purified water was added to prepare a binding solution with a concentration of 10% by mass.

### Example 1A

Ferric citrate (450 g, equivalent to anhydride 383.9 g) was placed in a tumbling fluid bed dryer granulator (MP-01, manufactured by POWREX), granulated while spraying the binding solution (135 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder a".
The obtained "sieved powder a" (618 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) at compression rate 20 mm/min, compression pressure 3, 4, 5 or 7.5 kN to give a 12 mmϕ round-shaped tablet.

### Example 2A

Ferric citrate (450 g, equivalent to anhydride 383.9 g) was placed in a tumbling fluid bed dryer granulator (MP-01, manufactured by POWREX), granulated while spraying the binding solution (270 g) of Reference Example 1-2, at an air volume set such that ferric citrate had an appropriate fluidized state, at a charge air temperature of 60°C and at a rate of 4 - 5 g/min, and then dried at a charge air temperature of 60°C to give dried granules. The obtained dried granules were sieved with a stainless sieve (aperture 500 µm) to give "sieved powder b".
The obtained "sieved powder b" (618 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) at compression rate 20 mm/min, compression pressure 5, 7 or 10 kN to give a 12 mmϕ round-shaped tablet.

### Example 3A

Ferric citrate (450 g, equivalent to anhydride 383.9 g) was placed in a tumbling fluid bed dryer granulator (MP-01, manufactured by POWREX), granulated while spraying the binding solution (315 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder C".
The obtained "sieved powder c" (642 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) at compression rate 20 mm/min, compression pressure 4, 5 or 7.5 kN to give a 12 mmϕ round-shaped tablet.

### Example 4A

Ferric citrate (450 g, equivalent to anhydride 383.9 g) was placed in a tumbling fluid bed dryer granulator (MP-01, manufactured by POWREX), granulated while spraying the binding solution (630 g) of Reference Example 1-3, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder d".
The obtained "sieved powder d" (642 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) at compression rate 20 mm/min, compression pressure 3, 4 or 5 kN to give a 12 mmϕ round-shaped tablet.

### Experimental Example 1A (moldability test)

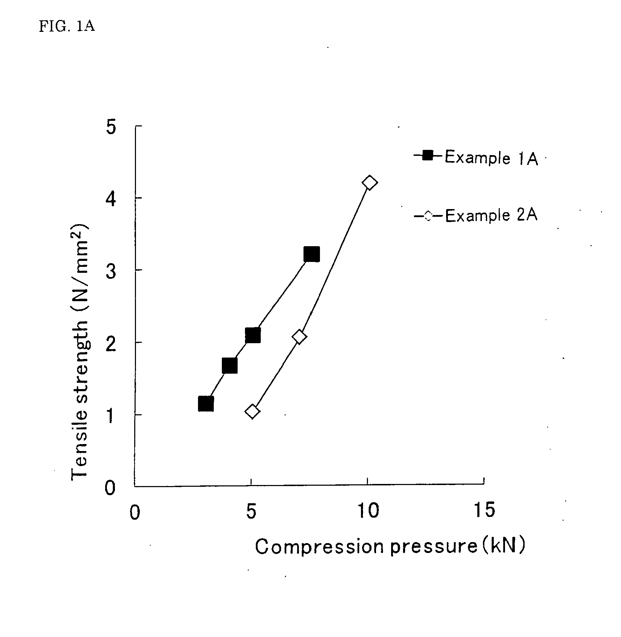

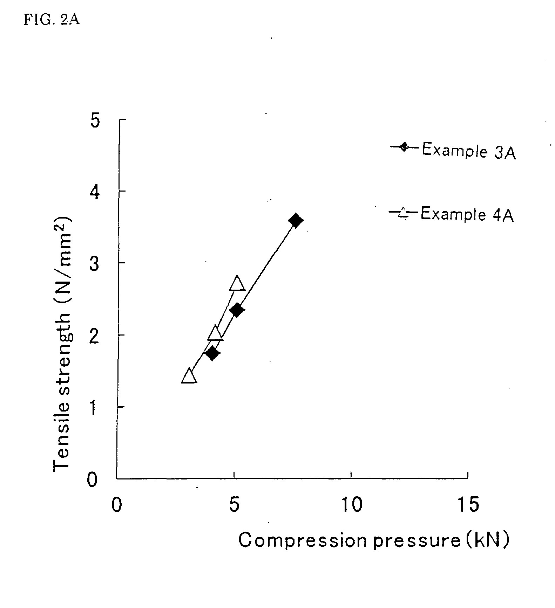

To examine tensile strength (hardness/fracture area) under each compression pressure of a tablet, the tablets obtained in Examples 1A - 4A were measured for tablet hardness (N) by a tablet hardness meter (6D, manufactured by Schleuniger), and the obtained values were divided by fracture area (tablet diameter (mm) x tablet thickness (mm)) to calculate tensile strength.
The tensile strength (N/mm²) was plotted relative to compression pressure (kN), and the results are shown in Figs. 1A and 2A.

### Example 5A

The "sieved powder a" (618 mg) obtained in Example 1A and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a compression pressure such that compression rate was 20 mm/min and tensile strength was about 2N/mm² to give a 12 mmϕ round-shaped tablet (containing 18 mg of partially pregelatinized starch per tablet).

### Example 6A

The "sieved powder b" (618 mg) obtained in Example 2A and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a compression pressure such that compression rate was 20 mm/min and tensile strength was about 2N/mm² to give a 12 mmϕ round-shaped tablet (containing 18 mg of hydroxypropylcellulose per tablet).

### Example 7A

The "sieved powder c" (642 mg) obtained in Example 3A and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a condition of a compression pressure such that compression rate was 20 mm/min and tensile strength was about 2N/mm² to give a 12 mmϕ round-shaped tablet (containing 42 mg of partially pregelatinized starch per tablet).

### Example 8A

The "sieved powder d" (642 mg) obtained in Example 4A and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a compression pressure such that compression rate was 20 mm/min and tensile strength was about 2N/mm² to give a 12 mmϕ round-shaped tablet (containing 42 mg of pregelatinized starch per tablet).

### Experimental Example 2A (dissolution test)

The tablets obtained in Examples 5A - 8A were subjected to a dissolution test under conditions shown in Table 1A, and dissolution property was evaluated.

**[Table 1A]**

| items | conditions |
|---|---|
| sample amount | 1 tablet (containing 600 mg of ferric citrate [equivalent to anhydride 511.8 mg] per tablet) |
| test method | The Japanese Pharmacopoeia 15, General Tests "Dissolution Test Method", Paddle Method Paddle rotation speed: 100 rpm |
| test solution | The Japanese Pharmacopoeia 15, Dissolution Test 1st fluid, 500 mL |
| dissolution test apparatus | automatic dissolution test apparatus (NTR-VS6P or NTR-6100, manufactured by Toyama Sangyo Co. Ltd.) |
| detector | ultraviolet visible spectrophotometer (UV-1600 or UV-1700, manufactured by SHIMADZU Corporation) |
| measurement wavelength | 334 nm and 600 nm |

In the Table, The Japanese Pharmacopoeia 15 shows the Japanese Pharmacopoeia, 15th Edition.

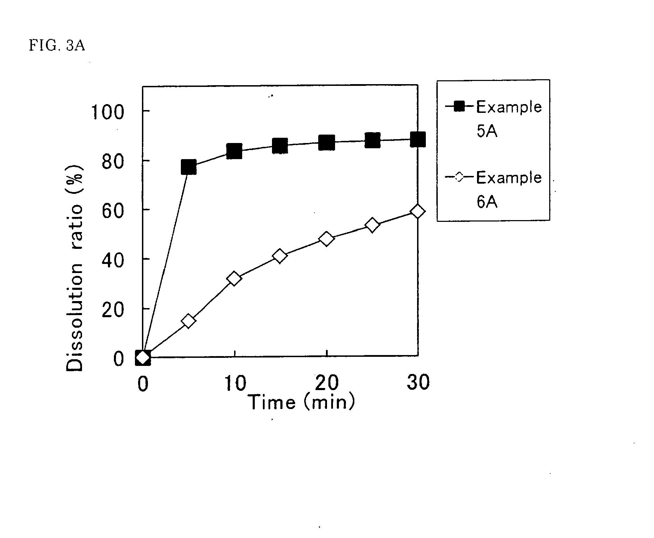

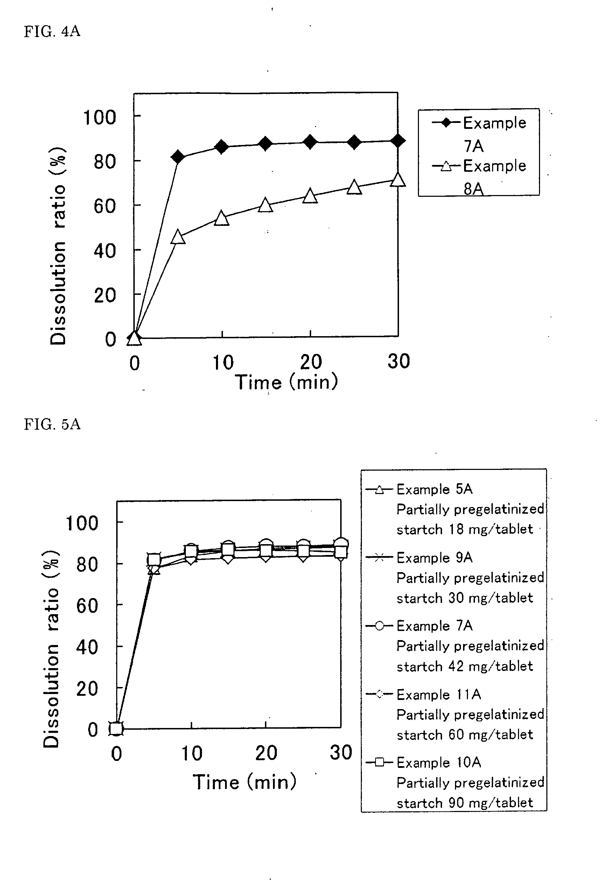

The results are shown in Figs. 3A and 4A.

### Example 9A

Ferric citrate (450 g, equivalent to anhydride 383.9 g) was placed in a tumbling fluid bed dryer granulator (MP-01, manufactured by POWREX), granulated while spraying the binding solution (225 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder e".
The obtained "sieved powder e" (630 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a compression pressure such that compression rate was 20 mm/min and tensile strength was about 2N/mm² to give a 12 mmϕ round-shaped tablet (containing 30 mg of partially pregelatinized starch per tablet).

### Example 10A

Ferric citrate (450 g, equivalent to anhydride 383.9 g) was placed in a tumbling fluid bed dryer granulator (MP-01, manufactured by POWREX), granulated while spraying the binding solution (675 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder f".
The obtained "sieved powder f" (690 mg) and magnesium stearate (Japanese Pharmacopoeia, plant-derived, manufactured by Taihei Chemical Industrial Co., Ltd.) as an external lubricant were molded by a 50 kN screw drive universal testing machine (SC-50HJ, manufactured by Tokyo Testing Machine) under a compression pressure such that compression rate was 20 mm/min and tablet hardness was about 140N to give a capsule-shaped tablet having major axis 17.5 mm, minor axis 8 mm (containing 90 mg of partially pregelatinized starch per tablet).

### Example 11A

Ferric citrate (450 g, equivalent to anhydride 383.9 g) was placed in a tumbling fluid bed dryer granulator (MP-01, manufactured by POWREX), granulated while spraying the binding solution (450 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 500 µm) to give "sieved powder g" (483.9 g).
The obtained "sieved powder g" (462 g) and magnesium stearate (4.76 g, Japanese Pharmacopoeia, plant-derived, manufactured by Taihei Chemical Industrial Co., Ltd.) were mixed in a 2 L container using a turbular mixer for 3 min to give a powder for tableting. This powder for tableting was molded by a rotary tableting machine (Correct 12HUK, manufactured by Kikusui Seisakusho Ltd.) under a compression pressure such that tablet hardness was 140 - 150N to give capsule-shaped tablets having major axis 17.5 mm, minor axis 8 mm (containing 60 mg of partially pregelatinized starch per tablet).

The component compositions of the tablets of Examples 1A - 4A, 6A and 8A are shown in Table 2A. In addition, ferric citrate (anhydride equivalent) content (%) of the plain tablet and the mass of partially pregelatinized starch, hydroxypropylcellulose and pregelatinized starch relative to 100 parts by mass of ferric citrate (anhydride equivalent) are shown in Table 3A.

**[Table 2A]**

| components | Example 1A | Example 2A | Example 3A | Example 4A | Example 6A | Example 8A |
|---|---|---|---|---|---|---|
| ferric citrate | 600.0 | 600.0 | 600.0 | 600.0 | 600.0 | 600.0 |
| ferric citrate (anhydride equivalent) | (511.8) | (511.8) | (511.8) | (511.8) | (511.8) | (511.8) |
| partially pregelatinized starch | 18.0 | --- | 42.0 | --- | --- | --- |
| hydroxypropylcellulose | --- | 18.0 | --- | --- | 18.0 | --- |
| pregelatinized starch | --- | --- | --- | 42.0 | --- | 42.0 |
| magnesium stearate | * | * | * | * | * | * |
| total tablet (mass containing water content of ferric citrate) | 618.0 | 618.0 | 642.0 | 642.0 | 618.0 | 642.0 |
| total tablet (mass without water content of ferric citrate) | 529.8 | 529.8 | 553.8 | 553.8 | 529.8 | 553.8 |

| | | | | | | |
|---|---|---|---|---|---|---|
| * : When magnesium stearate was used as an external lubricant, the amount was deemed as zero. The numerical values in the Table show ingredients (mg) per tablet. | | | | | | |

**[Table 3A]**

| | Example 1A | Example 2A | Example 3A | Example 4A | Example 6A | Example 8A |
|---|---|---|---|---|---|---|
| ferric citrate (anhydride equivalent) content (%) of plain tablet (*1) | 96.6 | 96.6 | 92.4 | 92.4 | 96.6 | 92.4 |
| mass of partially pregelatinized starch, hydroxypropylcellulose or Pregelatinized starch relative to 100 parts by mass of ferric citrate (anhydride equivalent) (*2) | 3.5 | 3.5 | 8.2 | 8.2 | 3.5 | 8.2 |

| | | | | | | |
|---|---|---|---|---|---|---|
| *1: [ferric citrate (anhydride equivalent)/total tablet (mass without water content of ferric citrate)]x100 *2: [partially pregelatinized starch, hydroxypropylcellulose or pregelatinized starch/ferric citrate (anhydride equivalent)]x100 | | | | | | |

The component compositions of the tablets of Examples 5A, 7A, 9A - 11A are shown in Table 4A. In addition, ferric citrate (anhydride equivalent) content (%) of the plain tablet and the mass of partially pregelatinized starch relative to 100 parts by mass of ferric citrate (anhydride equivalent) are shown in Table 5A.

**[Table 4A]**

| components | Example 5A | Example 9A | Example 7A | Example 11A | Example 10A |
|---|---|---|---|---|---|
| ferric citrate | 600.0 | 600.0 | 600.0 | 600.0 | 600.0 |
| ferric citrate (anhydride equivalent) | (511.8) | (511.8) | (511.8) | (511.8) | (511.8) |
| partially pregelatinized starch | 18.0 | 30.0 | 42.0 | 60.0 | 90.0 |
| magnesium stearate | * | * | * | 6.8 | * |
| total tablet (mass containing water content of ferric citrate) | 618.0 | 630.0 | 642.0 | 666.8 | 690.0 |
| total tablet (mass without water content of ferric citrate) | 529.8 | 541.8 | 553.8 | 578.6 | 601.8 |

| | | | | | |
|---|---|---|---|---|---|
| *: When magnesium stearate was used as an external lubricant, the amount was deemed to be zero. The numerical values in the Table show ingredients (mg) per tablet. | | | | | |

**[Table 5A]**

| | Example 5A | Example 9A | Example 7A | Example 11A | Example 10A |
|---|---|---|---|---|---|
| ferric citrate (anhydride equivalent) content (%) in plain tablet (*1) | 96.6 | 94.5 | 92.4 | 88.5 | 85.0 |
| mass of partially pregelatinized starch relative to 100 parts by mass of ferric citrate (anhydride equivalent) (*2) | 3.5 | 5.9 | 8.2 | 11.7 | 17.6 |

| | | | | | |
|---|---|---|---|---|---|
| *1: [ferric citrate (anhydride equivalent)/total tablet (mass without water content of ferric citrate)]x100 *2: [partially pregelatinized starch/ferric citrate (anhydride equivalent)]x100 | | | | | |

### Experimental Example 3A

The tablets obtained in Examples 5A, 7A, 9A, 10A and 11A were subjected to a dissolution test under conditions shown in Table 1A of Experimental Example 2A, and dissolution property was evaluated. The results of the dissolution test are shown in Fig. 5A.

### Example 12A

Ferric citrate (19.2757 kg, equivalent to anhydride 16.5 kg) was placed in a fluid bed dryer granulator (WSG-60, manufactured by POWREX), granulated while spraying the binding solution (19800 g) of Reference Example 1-4, at an air volume set such that ferric citrate had an appropriate fluidized state, at a charge air temperature of 70°C and at a rate of 170 - 200 g/min, and then dried at a charge air temperature of 70°C to give dried granules. The obtained dried granules were sieved with a screen (aperture 1143 µm) by a screen mill (U20, manufactured by POWREX).
The above-mentioned operation was repeated 3 times, and the granules were mixed to give "sieved powder h". To the obtained "sieved powder h" (62.0441 kg) was added calcium stearate (1300.4 g), they were mixed, and the obtained mixture was molded by a rotary tableting machine (Correct 12HUK, manufactured by Kikusui Seisakusho Ltd.) under a compression pressure such that tablet hardness was not less than 150N to give plain tablets having major axis 17.5 mm, minor axis 8 mm (capsule-shaped tablets).
The plain tablets (10.38 kg) were coated with a coating solution obtained by mixing opadry II (manufactured by Japan Colorcon, 1770 g), yellow ferric oxide (30 g), and purified water (10200 g), by an automatic coating machine (HCT-60N, manufactured by Freund Corporation) to give tablets having an about 30 mg of a coating layer per tablet.
The component composition of the tablet of Example 12A is shown in Table 6A. In addition, ferric citrate (anhydride equivalent) content (%) of the plain tablet and the mass of partially pregelatinized starch relative to 100 parts by mass of ferric citrate (anhydride equivalent) are shown in Table 7A.

**[Table 6A]**

| components | Example 12A |
|---|---|
| ferric citrate | 584.1 |
| ferric citrate (anhydride equivalent) | (500.0) |
| partially pregelatinized starch | 60.0 |
| calcium stearate | 13.5 |
| total plain tablet (mass containing water content of ferric citrate) | 657.6 |
| total plain tablet (mass without water content of ferric citrate) | 573.5 |
| opadry II | 29.5 |
| yellow ferric oxide | 0.5 |
| total coating films | 30.0 |

| | |
|---|---|
| The numerical values in the Table show ingredients (mg) per tablet. | |

**[Table 7A]**

| | Example 12A |
|---|---|
| ferric citrate (anhydride equivalent) content (%) of the plain tablet (*1) | 87.2 |
| mass of partially pregelatinized starch relative to 100 parts by mass of ferric citrate (anhydride equivalent) (*2) | 12.0 |

| | |
|---|---|
| *1: [ferric citrate (anhydride equivalent)/total plain tablet (mass without water content of ferric citrate)]x100 *2: [partially pregelatinized starch/ferric citrate (anhydride equivalent)]x100 | |

### Experimental Example 4A

The tablet (coated tablet) obtained in Example 12A was subjected to a dissolution test under the conditions shown in Table 8A, and the dissolution property was evaluated.

**[Table 8A]**

| items | conditions |
|---|---|
| sample amount | 1 tablet (containing ferric citrate equivalent to anhydride 500 mg per tablet) |
| test method | The Japanese Pharmacopoeia 15, General Tests "Dissolution Test Method", Paddle Method, Paddle rotating speed: 100 rpm |
| test solution | The Japanese Pharmacopoeia 15, Dissolution Test, 2nd fluid, 900 mL |
| dissolution test apparatus | automatic dissolution test apparatus (NTR-VS6P, manufactured by Toyama Sangyo Co. Ltd.) |
| detector | ultraviolet visible spectrophotometer (UV-1700, manufactured by SHIMADZU Corporation) |
| measurement wavelength | 360 nm and 600 nm |

| | |
|---|---|
| In the Table, The Japanese Pharmacopoeia 15 means the Japanese Pharmacopoeia, 15th Edition. | |

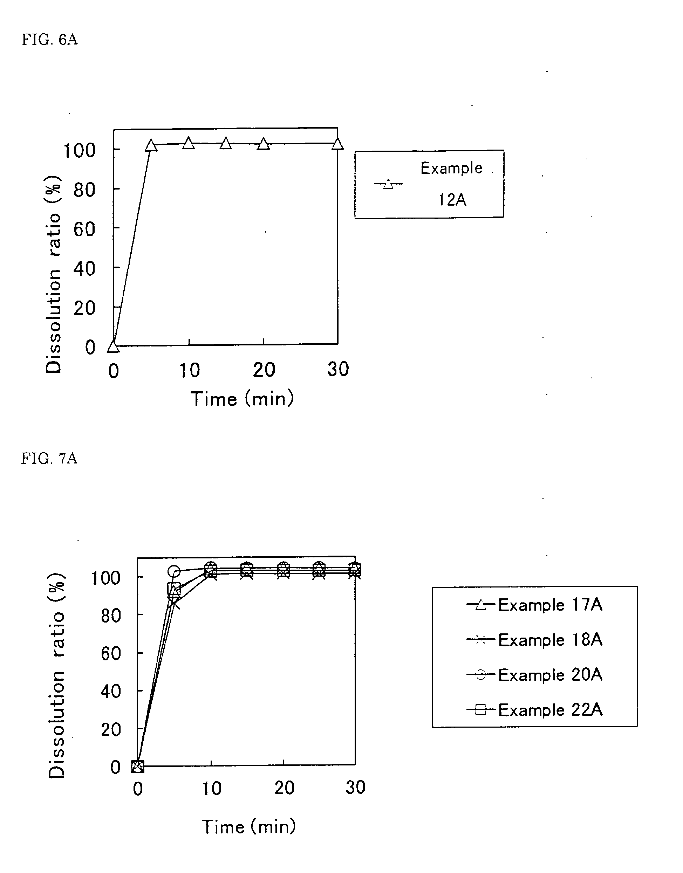

The results are shown in Fig. 6A.

### Example 13A

Ferric citrate (17.667 kg, equivalent to anhydride 15 kg) was placed in a fluid bed dryer granulator (WSG-60, manufactured by POWREX), granulated while spraying the binding solution (18000 g) of Reference Example 1-4, at an air volume set such that ferric citrate had an appropriate fluidized state, at a charge air temperature of 70°C and at a rate of 120 - 170 g/min, and then dried at a charge air temperature of 70°C to give dried granules. The obtained dried granules were sieved with a screen (aperture 813 µm) by a screen mill (U20, manufactured by POWREX).
The above-mentioned operation was repeated twice, and granules were mixed to give "sieved powder i".
Calcium stearate (607.5 g) was added to and mixed with the obtained "sieved powder i" (27.9027 kg) to give "tableting powder j".
The obtained "tableting powder j" (662.4 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch per tablet).

### Example 14A

The "tableting powder j" (7.618 g) obtained in Example 13A and low-substituted hydroxypropylcellulose (LH-11, manufactured by Shin-Etsu Chemical Co., Ltd., 0.345 g) were mixed in a 50 mL container for 3 min by using a turbular mixer to give "tableting powder k".
This "tableting powder k" (692.4 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch and 30 mg of low-substituted hydroxypropylcellulose per tablet).

### Example 15A

The "tableting powder j" (7.618 g) obtained in Example 13A and crystalline cellulose (CEOLUS KG-1000, manufactured by Asahikasei Chemicals, 0.690 g) were mixed in a 50 mL container for 3 min by using a turbular mixer to give "tableting powder l".
This "tableting powder 1" (722.4 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch and 60 mg of crystalline cellulose per tablet).

The component compositions of the tablets of Examples 13A - 15A are shown in Table 9A. In addition, ferric citrate (anhydride equivalent) content (%) of the plain tablet, the mass of partially pregelatinized starch relative to 100 parts by mass of ferric citrate (anhydride equivalent) and the mass of low-substituted hydroxypropylcellulose and crystalline cellulose relative to 100 parts by mass of ferric citrate (anhydride equivalent) are shown in Table 10A.

**[Table 9A]**

| components | Example 13A | Example 14A | Example 15A |
|---|---|---|---|
| ferric citrate | 588.9 | 588.9 | 588.9 |
| ferric citrate (anhydride equivalent) | (500.0) | (500.0) | (500.0) |
| partially pregelatinized starch | 60.0 | 60.0 | 60.0 |
| low-substituted hydroxypropylcellulose | --- | 30.0 | --- |
| crystalline cellulose | --- | --- | 60.0 |
| calcium stearate | 13.5 | 13.5 | 13.5 |
| total tablet (mass containing water content of ferric citrate) | 662.4 | 692.4 | 722.4 |
| total tablet (mass without water content of ferric citrate) | 573.5 | 603.5 | 633.5 |

| | | | |
|---|---|---|---|
| The numerical values in the Table show ingredients (mg) per tablet. | | | |

**[Table 10A]**

| | Example 13A | Example 14A | Example 15A |
|---|---|---|---|
| ferric citrate (anhydride equivalent) content (%) of the plain tablet (*1) | 87.2 | 82.8 | 78.9 |
| mass of partially pregelatinized starch relative to 100 parts by mass of ferric citrate (anhydride equivalent) (*2) | 12.0 | 12.0 | 12.0 |
| mass of low-substituted hydroxypropylcellulose and crystalline cellulose relative to 100 parts by mass of ferric citrate (anhydride equivalent) (*3) | --- | 6.0 | 12.0 |

| | | | |
|---|---|---|---|
| *1: [ferric citrate (anhydride equivalent/total tablet (mass without water content of ferric citrate)]x100 *2: [partially pregelatinized starch/ferric citrate (anhydride equivalent)]x100 *3: [low-substituted hydroxypropylcellulose and crystalline cellulose/ferric citrate (anhydride equivalent)]x100 | | | |

### Experimental Example 5A (moldability test)

The presence or absence of crack in the tablets obtained in Examples 13A - 15A was confirmed by visual examination by the naked eye. In addition, the tablet hardness was measured by a tablet hardness meter (6D, manufactured by Schleuniger). The results of the moldability test are shown in Table 11A.

**[Table 11A]**

| | Example 13A | Example 14A | Example 15A |
|---|---|---|---|
| rate of crack observed | 100% | 0% | 40% |
| tablet hardness | 96N | 166N | 144N |

### Example 16A

Ferric citrate (497.1 g, equivalent to anhydride 425 g) was placed in a tumbling fluid bed dryer granulator (MP-01, manufactured by POWREX), granulated while spraying the binding solution (510 g) of Reference Example 1-1, at an air volume set such that ferric citrate had an appropriate fluidized state, at a charge air temperature of 70°C and at a rate of 4 - 5 g/min, and then dried at a charge air temperature of 70°C to give dried granules. The obtained dried granules were sieved with a stainless sieve (aperture 710 µm) to give a sieved powder. The obtained sieved powder (10.3168 g) and low-substituted hydroxypropylcellulose (LH-11, manufactured by Shin-Etsu Chemical Co., Ltd., 0.96 g) were mixed in a 50 mL container for 5 min by using a turbular mixer. Calcium stearate (the Japanese Pharmacopoeia, plant-derived, manufactured by Taihei Chemical Industrial Co., Ltd., 0.216 g) was added and the mixture was admixed by using a turbular mixer for 3 min to give "tableting powder m".
This "tableting powder m" (718.3 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch and 60 mg of low-substituted hydroxypropylcellulose per tablet).

### Reference Example 2

Trehalose (trehalose P, manufactured by Asahikasei Chemicals, 35 g) and partially pregelatinized starch (Starch 1500G, manufactured by Japan Colorcon, 70 g) were dissolved or dispersed in purified water by using a propeller mixer, and purified water was added to a total amount of 700 g. The solution was sieved with a stainless sieve (aperture 250 µm) to prepare a binding solution.

### Example 17A

Ferric citrate (438.6 g, equivalent to anhydride 375 g) was placed in a tumbling fluid bed dryer granulator (MP-01, manufactured by POWREX), granulated while spraying the binding solution (450 g) of Reference Example 2, at an air volume set such that ferric citrate had an appropriate fluidized state, at a charge air temperature of 70°C and at a rate of 5 - 6.5 g/min, and then dried at a charge air temperature of 70°C to give dried granules. The obtained dried granules were sieved with a stainless sieve (aperture 500 µm) to give a sieved powder. The obtained sieved powder (10.7968 g) and low-substituted hydroxypropylcellulose (LH-11, manufactured by Shin-Etsu Chemical Co., Ltd., 0.48 g) were mixed in a 50 mL container for 5 min by using a turbular mixer. Calcium stearate (the Japanese Pharmacopoeia, plant-derived, manufactured by Taihei Chemical Industrial Co., Ltd., 0.216 g) was added and the mixture was admixed by using a turbular mixer for 3 min to give "tableting powder n".
This "tableting powder n" (718.3 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch, 30 mg of low-substituted hydroxypropylcellulose and 30 mg of trehalose per tablet).

### Reference Example 3

A polyvinyl alcohol-polyethylene glycol graft copolymer (Kollicoat IR, manufactured by BASF, 35 g) and partially pregelatinized starch (Starch 1500G, manufactured by Japan Colorcon, 70 g) were dissolved or dispersed in purified water by using a propeller mixer, and purified water was added to a total amount of 700 g. This solution was sieved with a stainless sieve (aperture 250 µm) to prepare a binding solution.

### Example 18A

Ferric citrate (448.1 g, equivalent to anhydride 375 g) was placed in a tumbling fluid bed dryer granulator (MP-01, manufactured by POWREX), granulated while spraying the binding solution (450 g) of Reference Example 3, at an air volume set such that ferric citrate had an appropriate fluidized state, at a charge air temperature of 70°C and at a rate of 5 - 6 g/min, and then dried at a charge air temperature of 70°C to give dried granules. The obtained dried granules were sieved with a stainless sieve (aperture 500 µm) to give a sieved powder. The obtained sieved powder (15.1228 g) and low-substituted hydroxypropylcellulose (LH-11, manufactured by Shin-Etsu Chemical Co., Ltd., 0.66 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give "tableting powder o".
This "tableting powder o" (730.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of partially pregelatinized starch, 30 mg of low-substituted hydroxypropylcellulose and 30 mg of a polyvinyl alcohol-polyethylene glycol graft copolymer per tablet).

### Reference Example 4

A polyvinyl alcohol-polyethylene glycol graft copolymer (Kollicoat IR, manufactured by BASF) was dissolved in purified water by using a propeller mixer, and purified water was added to prepare a binding solution with a concentration of 10% by mass.

### Example 19A

Ferric citrate (448.1 g, equivalent to anhydride 375 g) was placed in a tumbling fluid bed dryer granulator (MP-01, manufactured by POWREX), granulated while spraying the binding solution (225 g) of Reference Example 1-4, at an air volume set such that ferric citrate had an appropriate fluidized state, at a charge air temperature of 70°C and at a rate of 5 - 6 g/min, and then spraying the binding solution (225 g) of Reference Example 4, and further dried at a charge air temperature of 70°C to give dried granules. The obtained dried granules were sieved with a stainless sieve (aperture 500 µm) to give "sieved powder p".
The obtained "sieved powder p" (13.148 g) and low-substituted hydroxypropylcellulose (LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was further added and the mixture was admixed by using a turbular mixer for 3 min to give "tableting powder q".
This "tableting powder q" (730.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 30 mg of partially pregelatinized starch, 60 mg of low-substituted hydroxypropylcellulose and 30 mg of a polyvinyl alcohol-polyethylene glycol graft copolymer per tablet).

The component compositions of the tablets of Examples 16A - 19A are shown in Table 12A. In addition, ferric citrate (anhydride equivalent) content (%) of the plain tablet, the mass of partially pregelatinized starch relative to 100 parts by mass of ferric citrate (anhydride equivalent), the mass of low-substituted hydroxypropylcellulose and crystalline cellulose relative to 100 parts by mass of ferric citrate (anhydride equivalent), the mass of trehalose relative to 100 parts by mass of ferric citrate (anhydride equivalent) and the mass of a polyvinyl alcohol-polyethylene glycol graft copolymer relative to 100 parts by mass of ferric citrate (anhydride equivalent) are shown in Table 13A.

**[Table 12A]**

| components | Example 16A | Example 17A | Example 18A | Example 19A |
|---|---|---|---|---|
| ferric citrate | 584.8 | 584.8 | 597.4 | 597.4 |
| ferric citrate (anhydride equivalent) | (500.0) | (500.0) | (500.0) | (500.0) |
| partially pregelatinized starch | 60.0 | 60.0 | 60.0 | 30.0 |
| trehalose | --- | 30.0 | --- | --- |
| polyvinyl alcohol-polyethylene glycol graft copolymer | --- | --- | 30.0 | 30.0 |
| low-substituted hydroxypropylcellulose | 60.0 | 30.0 | 30.0 | 60.0 |
| crystalline cellulose | --- | --- | --- | --- |
| calcium stearate | 13.5 | 13.5 | 13.5 | 13.5 |
| total tablet (mass containing water content of ferric citrate) | 718.3 | 718.3 | 730.9 | 730.9 |
| total tablet (mass without water content of ferric citrate) | 633.5 | 633.5 | 633.5 | 633.5 |

| | | | | |
|---|---|---|---|---|
| The numerical values in the Table show ingredients (mg) per tablet. | | | | |

**[Table 13A]**

| | Example 16A | Example 17A | Example 18A | Example 19A |
|---|---|---|---|---|
| ferric citrate (anhydride equivalent) content (%) of the plain tablet (*1) | 78.9 | 78.9 | 78.9 | 78.9 |
| mass of partially pregelatinized starch relative to 100 parts by mass of ferric citrate (anhydride equivalent) (*2) | 12.0 | 12.0 | 12.0 | 6.0 |
| mass of trehalose relative to 100 parts by mass of ferric citrate (anhydride equivalent) (*3) | --- | 6.0 | --- | --- |
| mass of polyvinyl alcohol-polyethylene glycol graft copolymer relative to 100 parts by mass of ferric citrate (anhydride equivalent) (*4) | --- | --- | 6.0 | 6.0 |
| mass of low-substituted hydroxypropylcellulose and crystalline cellulose relative to 100 parts by mass of ferric citrate (anhydride equivalent) (*5) | 12.0 | 6.0 | 6.0 | 12.0 |

| | | | | |
|---|---|---|---|---|
| *1: [ferric citrate (anhydride equivalent)/total tablet (mass without water content of ferric citrate)]x100 *2: [partially pregelatinized starch/ferric citrate (anhydride equivalent)]x100 *3: [trehalose/ferric citrate (anhydride equivalent)]x100 *4: [polyvinyl alcohol-polyethylene glycol graft copolymer/ferric citrate (anhydride equivalent)]x100 *5: [low-substituted hydroxypropylcellulose and crystalline cellulose/ferric citrate (anhydride equivalent)]x100 | | | | |

### Experimental Example 6A (moldability test)

The tablets obtained in Examples 16A - 19A were confirmed for the presence or absence of cracks with a stereomicroscope (SZH, manufactured by OLYMPUS) at a magnification ratio of x15. In addition, tablet hardness was measured by a tablet hardness meter (6D, manufactured by Schleuniger).

### Experimental Example 7A (disintegration test)

The tablets obtained in Examples 16A - 19A were subjected to a disintegration test under the conditions shown in Table 14A, and disintegration property was evaluated.

**[Table 14A]**

| items | conditions |
|---|---|
| sample amount | 1 tablet (containing ferric citrate equivalent to anhydride 500 mg per tablet) |
| test method | The Japanese Pharmacopoeia 15, General Tests "disintegration test method" |
| test solution | The Japanese Pharmacopoeia 15, Disintegration Test 1st fluid |
| disks | none |
| disintegration test apparatus | automatic disintegration test apparatus (NT-2HS, manufactured by Toyama Sangyo Co. Ltd.). |

| | |
|---|---|
| In the Table, The Japanese Pharmacopoeia 15 means the Japanese Pharmacopoeia, 15th Edition. | |

The results of Experimental Examples 6A and 7A are shown in Table 15A.

**[Table 15A]**

| | Example 16A | Example 17A | Example 18A | Example 19A |
|---|---|---|---|---|
| rate of crack observed | 60% | 10% | 30% | 10% |
| tablet hardness | 195N | 227N | 192N | 215N |
| disintegration time | 0.8 min | 2.0 min | 4.0 min | 3.6 min |

### Example 20A

Ferric citrate (23.476 kg, equivalent to anhydride 20 kg) was placed in a fluid bed dryer granulator (WSG-60, manufactured by POWREX), granulated while spraying the binding solution (24000 g) of Reference Example 1-4, at an air volume set such that ferric citrate had an appropriate fluidized state, at a charge air temperature of 70°C and at a rate of 170 - 230 g/min, and then dried at a charge air temperature of 70°C to give dried granules. The obtained dried granules were sieved with a screen (aperture 1143 µm) by a screen mill (U20, manufactured by POWREX) to give "sieved powder r".
Ferric citrate (46.952 kg, equivalent to anhydride 40 kg) was placed in a fluid bed dryer granulator (WSG-60, manufactured by POWREX), granulated while spraying the binding solution (48000 g) of Reference Example 1-4, at an air volume set such that ferric citrate had an appropriate fluidized state, at a charge air temperature of 70°C and at a rate of 130 - 250 g/min, and then dried at a charge air temperature of 70°C to give dried granules. The obtained dried granules were sieved with a screen (aperture 1143 µm) by a screen mill (U20, manufactured by POWREX) to give "sieved powder s".
To the obtained "sieved powder r" (24.6610 kg) and "sieved powder s" (25.7972 kg) was added low-substituted hydroxypropylcellulose (LH-11, manufactured by Shin-Etsu Chemical Co., Ltd., 2340 g) and the mixture was stirred by a W-type blending machine (W-200, manufactured by Tokuju Corporation) at 26 rpm for 277 sec. Then, calcium stearate (the Japanese Pharmacopoeia, plant-derived, manufactured by Taihei Chemical Industrial Co., Ltd., 1053 g) was added, and the mixture was stirred by a W-type blending machine (W-200, manufactured by Tokuju Corporation) at 26 rpm for 92 sec to give a powder for tableting.
The obtained powder for tableting was molded by a rotary tableting machine (Correct 12HUK, manufactured by Kikusui Seisakusho Ltd.) under a compression pressure such that tablet hardness was not less than 150N to give a plain tablet having major axis 17.5 mm, minor axis 8 mm (capsule-shaped tablet).

### Example 21A

The plain tablets (11.0916 kg) obtained in Example 20A were coated with a coating solution obtained by mixing hypromellose (TC-5M, manufactured by Shin-Etsu Chemical Co., Ltd., 1080 g), titanium oxide (Titanium(IV) Oxide extra pure, manufactured by Merck, 360 g), talc (Hi-Filler #17, manufactured by Matsumura Sangyo Co. Ltd., 180 g), macrogol 6000 (macrogol 6000P, manufactured by NOF Corporation, 180 g), and purified water (12600 g), by an automatic coating machine (HCT-60N, manufactured by Freund Corporation) to give tablets having an about 25 mg of a coating layer per tablet.

### Example 22A

The "sieved powder p" (361.57 g) obtained in Example 19A and low-substituted hydroxypropylcellulose (LH-11, manufactured by Shin-Etsu Chemical Co., Ltd., 33.0 g) were mixed by a turbular mixer for 5 min. Calcium stearate (the Japanese Pharmacopoeia, plant-derived, manufactured by Taihei Chemical Industrial Co., Ltd., 7.425 g) was added, and the mixture was admixed by using a turbular mixer for 3 min to give a powder for tableting. This tableting powder was molded by a rotary tableting machine (Correct 12HUK, manufactured by Kikusui Seisakusho Ltd.) under a compression pressure such that tablet hardness was not less than 150N to give a plain tablet having major axis 17.5 mm, minor axis 8 mm (capsule-shaped tablet).

### Example 23A

The plain tablets (146.2 g) obtained in Example 22A were coated with a coating solution obtained by mixing hypromellose (TC-5M, manufactured by Shin-Etsu Chemical Co., Ltd., 30 g), titanium oxide (Titanium(IV) Oxide extra pure, manufactured by Merck, 10 g), talc (Hi-Filler #17, manufactured by Matsumura Sangyo Co. Ltd., 5 g), macrogol 6000 (macrogol 6000P, manufactured by NOF Corporation, 5 g), and purified water (350 g), by an automatic coating machine (HC-LABO, manufactured by Freund Corporation) to give tablets having an about 25 mg of a coating layer per tablet.
The component compositions of the tablets of Examples 20A - 23A are shown in Table 16A. In addition, ferric citrate (anhydride equivalent) content (%) of the plain tablet, the mass of partially pregelatinized starch relative to 100 parts by mass of ferric citrate (anhydride equivalent), the mass of a polyvinyl alcohol-polyethylene glycol graft copolymer relative to 100 parts by mass of ferric citrate (anhydride equivalent) and the mass of low-substituted hydroxypropylcellulose and crystalline cellulose relative to 100 parts by mass of ferric citrate (anhydride equivalent) are shown in Table 17A.

**[Table 16A]**

| components | Example 20A | Example 21A | Example 22A | Example 23A |
|---|---|---|---|---|
| ferric citrate | 586.9 | 586.9 | 597.4 | 597.4 |
| ferric citrate (anhydride equivalent) | (500.0) | (500.0) | (500.0) | (500.0) |
| partially Pregelatinized starch | 60.0 | 60.0 | 30.0 | 30.0 |
| polyvinyl alcohol-polyethylene glycol graft copolymer | --- | --- | 30.0 | 30.0 |
| low-substituted hydroxypropylcellulose | 30.0 | 30.0 | 60.0 | 60.0 |
| crystalline cellulose | --- | --- | --- | --- |
| calcium stearate | 13.5 | 13.5 | 13.5 | 13.5 |
| total tablet (mass containing water content of ferric citrate) | 690.4 | 690.4 | 730.9 | 730.9 |
| total tablet (mass without water content of ferric citrate) | 603.5 | 603.5 | 633.5 | 633.5 |
| hypromellose | --- | 15.0 | --- | 15.0 |
| titanium oxide | --- | 5.0 | --- | 5.0 |
| talc | --- | 2.5 | --- | 2.5 |
| macrogol 6000 | --- | 2.5 | --- | 2.5 |
| total coating film | --- | 25.0 | --- | 25.0 |

| | | | | |
|---|---|---|---|---|
| The numerical values in the Table show ingredients (mg) per tablet. | | | | |

**[Table 17A]**

| | Example 20A | Example 21A | Example 22A | Example 23A |
|---|---|---|---|---|
| ferric citrate (anhydride equivalent) content (%) of the plain tablet (*1) | 82.9 | 82.9 | 78.9 | 78.9 |
| mass of partially pregelatinized starch relative to 100 parts by mass of ferric citrate (anhydride equivalent) (*2) | 12.0 | 12.0 | 6.0 | 6.0 |
| mass of a polyvinyl alcohol-polyethylene glycol graft copolymer relative to 100 parts by mass of ferric citrate (anhydride equivalent) (*3) | --- | --- | 6.0 | 6.0 |
| mass of low-substituted hydroxypropylcellulose and crystalline cellulose relative to 100 parts by mass of ferric citrate (anhydride equivalent) (*4) | 6.0 | 6.0 | 12.0 | 12.0 |

| | | | | |
|---|---|---|---|---|
| *1: [ferric citrate (anhydride equivalent)/total tablet (mass without water content of ferric citrate)]x100 *2: [partially pregelatinized starch/ferric citrate (anhydride equivalent)]x100 *3: [polyvinyl alcohol-polyethylene glycol graft copolymer/ferric citrate (anhydride equivalent)]x100 *4: [low-substituted hydroxypropylcellulose and crystalline cellulose/ferric citrate (anhydride equivalent)]x100 | | | | |

### Experimental Example 8A

The tablets (plain tablets) obtained in Examples 17A, 18A, 20A and 22A were subjected to a dissolution test under the conditions shown in Table 8A, and the dissolution property was evaluated.
The results are shown in Fig. 7A.

### Experimental Example 9A

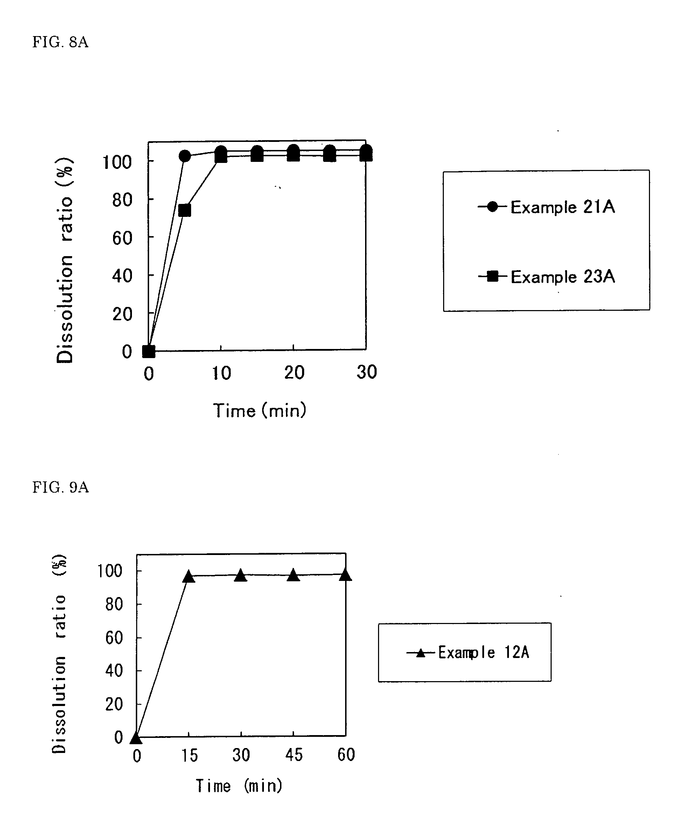

The tablets (coated tablets) obtained in Examples 21A and 23A were subjected to a dissolution test under the conditions shown in Table 8A, and the dissolution property was evaluated.
The results are shown in Fig. 8A.

### Experimental Example 10A

The tablet (coated tablet) obtained in Examples 12A was subjected to a dissolution test under the conditions shown in Table 18A, and the dissolution property was evaluated.

**[Table 18A]**

| | | |
|---|---|---|
| sample amount | | 1 tablet (containing ferric citrate equivalent to anhydride 500 mg per tablet) |
| method | | The Japanese Pharmacopoeia 15, General Tests, Dissolution Test Method 2nd method (Paddle Method) |
| conditions | Paddle rotation number | 50 rpm |
| | test solution | The Japanese Pharmacopoeia 15, Dissolution Test 2nd fluid, 900 mL |
| | ferric citrate quantification method | Color is developed with 1,10-phenanthrolin, and absorbance is measured. |
| dissolution test apparatus | | automatic dissolution test apparatus (NTR-6100A manufactured by Toyama Sangyo Co., Ltd.) |

| | | |
|---|---|---|
| In the Table, the Japanese Pharmacopoeia 15 means the Japanese Pharmacopoeia, 15th Edition. | | |

The results are shown in Fig. 9A.

### Example 1B

Hydroxypropylcellulose (HPC L, manufactured by Nippon Soda Co., Ltd.) was dissolved in purified water by using a propeller mixer to prepare a binding solution with a concentration of 5% by mass.
Ferric citrate (448.1 g, equivalent to anhydride 375 g) was placed in a tumbling fluid bed dryer granulator (MP-01, manufactured by POWREX), granulated while spraying the above-mentioned binding solution (450 g), and dried. The obtained dried granules were sieved with a stainless sieve (aperture 500 µm) to give a sieved powder. The sieved powder (13.8028 g) and low-substituted hydroxypropylcellulose (L-HPC LH-11, manufactured by Shin-Etsu Chemical Co., Ltd., 1.32 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 30 mg of hydroxypropylcellulose and 60 mg of low-substituted hydroxypropylcellulose per tablet).

### Example 2B

Hypromellose (TC-5R, manufactured by Shin-Etsu Chemical Co., Ltd.) was dissolved in purified water by using a propeller mixer to prepare a binding solution with a concentration of 5% by mass.
Ferric citrate (448.1 g, equivalent to anhydride 375 g) was placed in a tumbling fluid bed dryer granulator (MP-01, manufactured by POWREX), granulated while spraying the above-mentioned binding solution (450 g), and dried. The obtained dried granules were sieved with a stainless sieve (aperture 500 µm) to give a sieved powder. The sieved powder (13.8028 g and low-substituted hydroxypropylcellulose (L-HPC LH-11, manufactured by Shin-Etsu Chemical Co., Ltd., 1.32 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 30 mg of hypromellose and 60 mg of low-substituted hydroxypropylcellulose per tablet).

### Example 3B

Polyvinylpyrrolidone K30 (Kollidon 30, manufactured by BASF) was dissolved in purified water by using a propeller mixer to prepare a binding solution with a concentration of 10% by mass.
Ferric citrate (448.1 g, equivalent to anhydride 375 g) was placed in a tumbling fluid bed dryer granulator (MP-01, manufactured by POWREX), granulated while spraying the above-mentioned binding solution (450 g), and dried. The obtained dried granules were sieved with a stainless sieve (aperture 500 µm) to give a sieved powder. The sieved powder (14.4628 g and low-substituted hydroxypropylcellulose (L-HPC LH-11, manufactured by Shin-Etsu Chemical Co., Ltd., 1.32 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (730.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of polyvinylpyrrolidone K30 and 60 mg of low-substituted hydroxypropylcellulose per tablet).

### Example 4B

Polyvinylpyrrolidone K90 (Kollidon 90F, manufactured by BASF) was dissolved in purified water by using a propeller mixer to prepare a binding solution with a concentration of 4% by mass.
Ferric citrate (448.1 g, equivalent to anhydride 375 g) was placed in a tumbling fluid bed dryer granulator (MP-01, manufactured by POWREX), granulated while spraying the above-mentioned binding solution (562.5 g), and dried. The obtained dried granules were sieved with a stainless sieve (aperture 500 µm) to give a sieved powder. The sieved powder (12.548g and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 30 mg of polyvinylpyrrolidone K90 and 60 mg of low-substituted hydroxypropylcellulose per tablet).

### Example 5B

Pregelatinized starch (Amycol C, manufactured by NIPPON STARCH CHEMICAL CO., LTD.) was dispersed in purified water by using a propeller mixer, and purified water was added to prepare a binding solution with a concentration of 5% by mass, and sieved with a stainless sieve (aperture 250 µm).
Ferric citrate (438.6 g, equivalent to anhydride 375 g) was placed in a tumbling fluid bed dryer granulator (MP-01, manufactured by POWREX) and granulated while spraying the above-mentioned binding solution (450 g) and dried. The obtained dried granules were sieved with a stainless sieve (aperture 710 µm) to give a sieved powder. The sieved powder (9.8368 g and low-substituted hydroxypropylcellulose (L-HPC LH-11, manufactured by Shin-Etsu Chemical Co., Ltd., 0.96 g) were mixed in a 50 mL container for 5 min by using a turbular mixer. Calcium stearate (the Japanese Pharmacopoeia, plant-derived, manufactured by Taihei Chemical Industrial Co., Ltd., 0.216 g) was added and the mixture was admixed by using a turbular mixer for 3 min to give a powder for tableting. The obtained powder for tableting (688.3 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 30 mg of pregelatinized starch and 60 mg of low-substituted hydroxypropylcellulose per tablet).

### Example 6B

Polyvinyl alcohol-polyethylene glycol graft copolymer (Kollicoat IR, manufactured by BASF) was dissolved in purified water by using a propeller mixer to prepare a binding solution with a concentration of 15% by mass.
Ferric citrate (497.1 g, equivalent to anhydride 425 g) was placed in a tumbling fluid bed dryer granulator (MP-01, manufactured by POWREX) and granulated while spraying the above-mentioned binding solution (340 g) and dried. The obtained dried granules were sieved with a stainless sieve (aperture 710 µm) to give a sieved powder. The sieved powder (14.1856 g and low-substituted hydroxypropylcellulose (L-HPC LH-11, manufactured by Shin-Etsu Chemical Co., Ltd., 1.32 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (718.3 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 60 mg of a polyvinyl alcohol-polyethylene glycol graft copolymer and 60 mg of low-substituted hydroxypropylcellulose per tablet).

### Example 7B

A polyvinyl alcohol-acrylic acid-methyl methacrylate copolymer (POVACOAT Type:F, manufactured by Daido Chemical Corporation) was dissolved in purified water by using a propeller mixer to prepare a binding solution with a concentration of 5% by mass.
Ferric citrate (448.1 g, equivalent to anhydride 375 g) was placed in a tumbling fluid bed dryer granulator (MP-01, manufactured by POWREX) and granulated while spraying the above-mentioned binding solution (270 g) and dried. The obtained dried granules were sieved with a stainless sieve (aperture 500 µm) to give a sieved powder. The sieved powder (12.308 g and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (688.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 18 mg of polyvinyl alcohol-acrylic acid-methyl methacrylate copolymer and 60 mg of low-substituted hydroxypropylcellulose per tablet).

The component compositions of the tablets of Examples 1B - 7B are shown in Table 1B. In addition, ferric citrate (anhydride equivalent) content (%) of the plain tablet, the mass of hydroxypropylcellulose, hypromellose, polyvinylpyrrolidone K30 and K90, pregelatinized starch, polyvinyl alcohol-polyethylene glycol graft copolymer or polyvinyl alcohol-acrylic acid-methyl methacrylate copolymer relative to 100 parts by mass of ferric citrate (anhydride equivalent), and the mass of low-substituted hydroxypropylcellulose relative to 100 parts by mass of ferric citrate (anhydride equivalent) are shown in Table 2B.

**[Table 1B]**

| components | Example 1B | Example 2B | Example 3B | Example 4B | Example 5B | Example 6B | Example 7B |
|---|---|---|---|---|---|---|---|
| ferric citrate | 597.4 | 597.4 | 597.4 | 597.4 | 584.8 | 584.8 | 597.4 |
| ferric citrate (anhydride equivalent) | (500.0) | (500.0) | (500.0) | (500.0) | (500.0) | (500.0) | (500.0) |
| hydroxypropylcellulose | 30.0 | --- | --- | --- | --- | --- | --- |
| hypromellose | --- | 30.0 | --- | --- | --- | --- | --- |
| polyvinylpyrrolidone K30 | --- | --- | 60.0 | --- | --- | --- | --- |
| polyvinylpyrrolidone K90 | --- | --- | --- | 30.0 | --- | --- | --- |
| pregelatinized starch | --- | --- | --- | --- | 30.0 | --- | --- |
| polyvinyl alcohol-polyethylene glycol graft copolymer | --- | --- | --- | --- | --- | 60.0 | --- |
| polyvinyl alcohol-acrylic acid-methyl methacrylate copolymer | --- | --- | --- | --- | --- | --- | 18.0 |
| low-substituted hydroxypropylcellulose | 60.0 | 60.0 | 60.0 | 60.0 | 60.0 | 60.0 | 60.0 |
| calcium stearate | 13.5 | 13.5 | 13.5 | 13.5 | 13.5 | 13.5 | 13.5 |
| total tablet (mass containing water content of ferric citrate) | 700.9 | 700.9 | 730.9 | 700.9 | 688.3 | 718.3 | 688.9 |
| total tablet (mass without water content of ferric citrate) | 603.5 | 603.5 | 633.5 | 603.5 | 603.5 | 633.5 | 591.5 |

| | | | | | | | |
|---|---|---|---|---|---|---|---|
| The numerical values in the Table show ingredients (mg) per tablet. | | | | | | | |

**[Table 2B]**

| | Example 1B | Example 2B | Example 3B | Example 4B | Example 5B | Example 6B | Example 7B |
|---|---|---|---|---|---|---|---|
| ferric citrate (anhydride equivalent) content (%) of the plain tablet (*1) | 82.9 | 82.9 | 78.9 | 82.9 | 82.9 | 78.9 | 84.5 |
| mass of hydroxypropylcellulose, hypromellose, polyvinylpyrrolidone (K30 and K90), pregelatinized starch, polyvinyl alcohol-polyethylene glycol graft copolymer or polyvinyl alcohol-acrylic acid-methyl methacrylate copolymer relative to 100 parts by mass of ferric citrate (anhydride equivalent) (*2) | 6.0 | 6.0 | 12.0 | 6.0 | 6.0 | 12.0 | 3.6 |
| mass of low-substituted hydroxypropylcellulose relative to 100 parts by mass of ferric citrate (anhydride equivalent) (*3) | 12.0 | 12.0 | 12.0 | 12.0 | 12.0 | 12.0 | 12.0 |

| | | | | | | | |
|---|---|---|---|---|---|---|---|
| *1: [ferric citrate (anhydride equivalent)/total tablet (mass without water content of ferric citrate)]x100 *2: [hydroxypropylcellulose, hypromellose, polyvinylpyrrolidone (K30 and K90), pregelatinized starch, polyvinyl alcohol-polyethylene glycol graft copolymer or polyvinyl alcohol-acrylic acid-methyl methacrylate copolymer/ferric citrate (anhydride equivalent)]x100 *3: [low-substituted hydroxypropylcellulose/ferric citrate (anhydride equivalent)]x100 | | | | | | | |

### Example 8B

A polyvinyl alcohol-polyethylene glycol graft copolymer (Kollicoat IR, manufactured by BASF, 21.0 g) and a polyvinyl alcohol-acrylic acid-methyl methacrylate copolymer (POVACOAT Type: F, manufactured by Daido Chemical Corporation, 14.0 g) were added to purified water (315.0 g), and dissolved by using a propeller mixer to prepare a binding solution.
Ferric citrate (448.1 g, equivalent to anhydride 375 g) was placed in a tumbling fluid bed dryer granulator (MP-01, manufactured by POWREX) and granulated while spraying the above-mentioned binding solution (225 g) and dried. The obtained dried granules were sieved with a stainless sieve (aperture 500 µm) to give a sieved powder. The sieved powder (12.548 g and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 18 mg of a polyvinyl alcohol-polyethylene glycol graft copolymer, 12 mg of polyvinyl alcohol-acrylic acid-methyl methacrylate copolymer and 60 mg of low-substituted hydroxypropylcellulose per tablet).

### Example 9B

A polyvinyl alcohol-polyethylene glycol graft copolymer (Kollicoat IR, manufactured by BASF, 6.0 g) and a polyvinyl alcohol-acrylic acid-methyl methacrylate copolymer (POVACOAT Type: F, manufactured by Daido Chemical Corporation, 12.0 g) were added to purified water (282.0 g), and dissolved by using a propeller mixer to prepare a binding solution.
Ferric citrate (448.1 g, equivalent to anhydride 375 g) was placed in a tumbling fluid bed dryer granulator (MP-01, manufactured by POWREX) and granulated while spraying the above-mentioned binding solution (225 g) and dried. The obtained dried granules were sieved with a stainless sieve (aperture 500 µm) to give a sieved powder. The sieved powder (12.308 g) and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (688.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 6 mg of a polyvinyl alcohol-polyethylene glycol graft copolymer, 12 mg of polyvinyl alcohol-acrylic acid-methyl methacrylate copolymer and 60 mg of low-substituted hydroxypropylcellulose per tablet).

### Example 10B

A polyvinyl alcohol-polyethylene glycol graft copolymer (Kollicoat IR, manufactured by BASF, 14.0 g) and a polyvinyl alcohol-acrylic acid-methyl methacrylate copolymer (POVACOAT Type: F, manufactured by Daido Chemical Corporation, 7.0 g) were added to purified water (329.0 g), and dissolved by using a propeller mixer to prepare a binding solution.
Ferric citrate (448.1 g, equivalent to anhydride 375 g) was placed in a tumbling fluid bed dryer granulator (MP-01, manufactured by POWREX) and granulated while spraying the above-mentioned binding solution (225 g) and dried. The obtained dried granules were sieved with a stainless sieve (aperture 500 µm) to give a sieved powder. The sieved powder (12.308 g) and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (688.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 12 mg of a polyvinyl alcohol-polyethylene glycol graft copolymer, 6 mg of polyvinyl alcohol-acrylic acid-methyl methacrylate copolymer and 60 mg of low-substituted hydroxypropylcellulose per tablet).

### Example 11B

A polyvinyl alcohol-polyethylene glycol graft copolymer (Kollicoat IR, manufactured by BASF, 21.0 g) and a polyvinyl alcohol-acrylic acid-methyl methacrylate copolymer (POVACOAT Type: F, manufactured by Daido Chemical Corporation, 7.0 g) were added to purified water (322.0 g), and dissolved by using a propeller mixer to prepare a binding solution.
Ferric citrate (448.1 g, equivalent to anhydride 375 g) was placed in a tumbling fluid bed dryer granulator (MP-01, manufactured by POWREX) and granulated while spraying the above-mentioned binding solution (225 g) and dried. The obtained dried granules were sieved with a stainless sieve (aperture 500 µm) to give a sieved powder. The sieved powder (31.07 g) and low-substituted hydroxypropylcellulose (L-HPC LH-11, manufactured by Shin-Etsu Chemical Co., Ltd., 3.0 g) were mixed in a 100 mL container for 5 min by using a turbular mixer. Calcium stearate (the Japanese Pharmacopoeia, plant-derived, manufactured by Taihei Chemical Industrial Co., Ltd., 0.675 g) was added and the mixture was admixed by using a turbular mixer for 3 min to give a powder for tableting. The obtained powder for tableting (694.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 18 mg of a polyvinyl alcohol-polyethylene glycol graft copolymer, 6 mg of polyvinyl alcohol-acrylic acid-methyl methacrylate copolymer and 60 mg of low-substituted hydroxypropylcellulose per tablet).

### Example 12B

A polyvinyl alcohol-polyethylene glycol graft copolymer (Kollicoat IR, manufactured by BASF, 28.0 g) and a polyvinyl alcohol-acrylic acid-methyl methacrylate copolymer (POVACOAT Type: F, manufactured by Daido Chemical Corporation, 7.0 g) were added to purified water (315.0 g), and dissolved by using a propeller mixer to prepare a binding solution.
Ferric citrate (448.1 g, equivalent to anhydride 375 g) was placed in a tumbling fluid bed dryer granulator (MP-01, manufactured by POWREX) and granulated while spraying the above-mentioned binding solution (225 g) and dried. The obtained dried granules were sieved with a stainless sieve (aperture 500 µm) to give a sieved powder. The sieved powder (12.548 g) and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of low-substituted hydroxypropylcellulose per tablet).

The component compositions of the tablets of Examples 8B - 12B are shown in Table 3B. In addition, ferric citrate (anhydride equivalent) content (%) of the plain tablet, the mass of a polyvinyl alcohol-polyethylene glycol graft copolymer or a polyvinyl alcohol-acrylic acid-methyl methacrylate copolymer relative to 100 parts by mass of ferric citrate (anhydride equivalent) and the mass of low-substituted hydroxypropylcellulose relative to 100 parts by mass of ferric citrate (anhydride equivalent) are shown in Table 4B.

**[Table 3B]**

| components | Example 8B | Example 9B | Example 10B | Example 11E | Example 12B |
|---|---|---|---|---|---|
| ferric citrate | 597.4 | 597.4 | 597.4 | 597.4 | 597.4 |
| ferric citrate (anhydride equivalent) | (500.0) | (500.0) | (500.0) | (500.0) | (500.0) |
| polyvinyl alcohol-polyethylene glycol graft copolymer | 18.0 | 6.0 | 12.0 | 18.0 | 24.0 |
| polyvinyl alcohol-acrylic acid-methyl methacrylate copolymer | 12.0 | 12.0 | 6.0 | 6.0 | 6.0 |
| low-substituted hydroxypropylcellulose | 60.0 | 60.0 | 60.0 | 60.0 | 60.0 |
| calcium stearate | 13.5 | 13.5 | 13.5 | 13.5 | 13.5 |
| total tablet (mass containing water content of ferric citrate) | 700.9 | 688.9 | 688.9 | 694.9 | 700.9 |
| total tablet (mass without water content of ferric citrate) | 603.5 | 591.5 | 591.5 | 597.5 | 603.5 |

| | | | | | |
|---|---|---|---|---|---|
| The numerical values in the Table show ingredients (mg) per tablet. | | | | | |

**[Table 4B]**

| | Example 8B | Example 9B | Example 10B | Example 11B | Example 12B |
|---|---|---|---|---|---|
| ferric citrate (anhydride equivalent) content (%) of the plain tablet (*1) | 82.9 | 84.5 | 84.5 | 83.7 | 82.9 |
| mass of polyvinyl alcohol-polyethylene glycol graft copolymer relative to 100 parts by mass of ferric citrate (anhydride equivalent) (*2) | 3.6 | 1.2 | 2.4 | 3.6 | 4.8 |
| mass of polyvinyl alcohol-acrylic acid-methyl methacrylate copolymer relative to 100 parts by mass of ferric citrate (anhydride equivalent) (*3) | 2.4 | 2.4 | 1.2 | 1.2 | 1.2 |
| mass of low-substituted hydroxypropylcellulose relative to 100 parts by mass of ferric citrate (anhydride equivalent) (*4) | 12.0 | 12.0 | 12.0 | 12.0 | 12.0 |

| | | | | | |
|---|---|---|---|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride equivalent)]x100 *4: [low-substituted hydroxypropylcellulose/ferric citrate (anhydride equivalent)]x100 | | | | | |

### Experimental Example 1B (apparent specific volume of sieved powder)

The sieved powders obtained in Examples 1B - 12B were poured into a 100 mL stainless measurement container until it overflowed. Then, the excess amount of the sieved powder deposited on the container was carefully removed and the mass of 100 mL of the sieved powder was measured.

### Experimental Example 2B (moldability test)

The tablets obtained in Examples 1B - 12B were observed under a stereomicroscope (SZH, manufactured by OLYMPUS) at magnification x15 to confirm the presence or absence of cracks. In addition, tablet hardness was measured by a tablet hardness meter (6D, manufactured by Schleuniger).

### Experimental Example 3B (disintegration test)

The tablets obtained in Examples 1B - 12B were subjected to a disintegration test under the conditions shown in Table 5B and the disintegration property was evaluated.

**[Table 5B]**

| items | conditions |
|---|---|
| sample amount | 1 tablet (containing ferric citrate equivalent to anhydride 500 mg per tablet) |
| test method | The Japanese Pharmacopoeia 15, General Tests "Disintegration Test Method" |
| test solution | The Japanese Pharmacopoeia 15, Disintegration Test 1st fluid |
| disks | none |
| disintegration test apparatus | automatic disintegration test apparatus (NT-2HS, manufactured by Toyama Sangyo Co. Ltd.) |

| | |
|---|---|
| In the Table, the Japanese Pharmacopoeia 15 means the Japanese Pharmacopoeia, 15th Edition. | |

The results of Experimental Examples 1B, 2B and 3B are shown in Table 6B.

**[Table 6B]**

| | Example 1B | Example 2B | Example 3B | Example 4B | Example 5B | Example 6B | Example 7B | Example 8B | Example 9B | Example 10B | Example 11B | Example 12B |
|---|---|---|---|---|---|---|---|---|---|---|---|---|
| apparent specific volume of sieved powder (mL/g) | 1-74 | 2.54 | 2.74 | 3.09 | 2.62 | 2.29 | 3.00 | 3.10 | 2.79 | 2.44 | 2.71 | 2.92 |
| tablet hardness (N) | 141 | 128 | 179 | 184 | 212 | 194 | 192 | 230 | 193 | 184 | 202 | 215 |
| rate of observed cracks (per 10 tablets) | 3/10 | 2/10 | 9/10 | 9/10 | 4/10 | 0/10 | 0/10 | 0/10 | 3/10 | 5/10 | 1/10 | 1/10 |
| disintegration time (min) | 1.4 | 2.6 | 4.2 | 30 | 0.7 | 6.4 | 1.6 | 4.3 | 1.5 | 1.2 | 2.9 | 3.8 |

### Example 13B

A polyvinyl alcohol-polyethylene glycol graft copolymer (Kollicoat IR, manufactured by BASF, 1600 g) and a polyvinyl alcohol-acrylic acid-methyl methacrylate copolymer (POVACOAT Type: F, manufactured by Daido Chemical Corporation, 400 g) were added to purified water (24667 g), and dissolved by using a propeller mixer to prepare a binding solution.
Ferric citrate (17922 g, equivalent to anhydride 15000 g) was placed in a fluid bed dryer granulator (WSG-30, manufactured by POWREX) and granulated while spraying the above-mentioned binding solution (12000 g) and dried. The obtained dried granules were sieved with a stainless sieve (aperture 500 µm) to give a sieved powder.

### Example 14B

A sieved powder (12.548 g) obtained in Example 13B and low-substituted hydroxypropylcellulose (L-HPC LH-11, manufactured by Shin-Etsu Chemical Co., Ltd., 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of low-substituted hydroxypropylcellulose per tablet).

### Example 15B

A sieved powder (13.8028 g) obtained in Example 13B and carboxymethylcellulose (NS-300, manufactured by Nichirin Chemical Corpration, 1.32 g) were mixed in a 50 mL container for 5 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of carboxymethylcellulose per tablet).

### Example 16B

A sieved powder (25.096 g) obtained in Example 13B, low-substituted hydroxypropylcellulose (L-HPC LH-11, manufactured by Shin-Etsu Chemical Co., Ltd., 1.68 g) and crystalline cellulose (CEOLUS KG-1000, manufactured by Asahikasei Chemicals) 0.72g were mixed in a 100 mL container for 5 min by using a turbular mixer. Calcium stearate (the Japanese Pharmacopoeia, plant-derived, manufactured by Taihei Chemical Industrial Co., Ltd., 0.54 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer, 42 mg of low-substituted hydroxypropylcellulose and 18 mg of crystalline cellulose per tablet) .

### Example 17B

A sieved powder (25.096 g) obtained in Example 13B, low-substituted hydroxypropylcellulose (L-HPC LH-11, manufactured by Shin-Etsu Chemical Co., Ltd., 1.20 g) and crystalline cellulose (CEOLUS KG-1000, manufactured by Asahikasei Chemicals, 1.20 g) were mixed in a 100 mL container for 5 min by using a turbular mixer. Calcium stearate (the Japanese Pharmacopoeia, plant-derived, manufactured by Taihei Chemical Industrial Co., Ltd., 0.54 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer, 30 mg of low-substituted hydroxypropylcellulose and 30 mg of crystalline cellulose per tablet).

### Example 18B

A sieved powder (12.548 g) obtained in Example 13B and crospovidone (Kollidon CL, manufactured by BASF, 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of crospovidone per tablet).

### Example 19B

A sieved powder (12.548 g) obtained in Example 13B and partially pregelatinized starch (Starch 1500G, manufactured by Japan Colorcon, 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of partially pregelatinized starch per tablet).

### Example 20B

A sieved powder (12.548 g) obtained in Example 13B and pregelatinized starch (SWELSTAR PD-1, manufactured by Asahikasei Chemicals, 1.2 g) were mixed in a 50 mL container for 5 min by using a turbular mixer. Calcium stearate (the Japanese Pharmacopoeia, plant-derived, manufactured by Taihei Chemical Industrial Co., Ltd., 0.27 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer and 60 mg of pregelatinized starch per tablet).
The component compositions of the tablets of Examples 14B - 20B are shown in Table 7B. In addition, ferric citrate (anhydride equivalent) content (%) of the plain tablet, the mass of a polyvinyl alcohol-polyethylene glycol graft copolymer relative to 100 parts by mass of ferric citrate (anhydride equivalent), the mass of a polyvinyl alcohol-acrylic acid-methyl methacrylate copolymer relative to 100 parts by mass of ferric citrate (anhydride equivalent), and the mass of low-substituted hydroxypropylcellulose, carboxymethylcellulose, crystalline cellulose, crospovidone, partially pregelatinized starch or pregelatinized starch relative to 100 parts by mass of ferric citrate (anhydride equivalent) are shown in Table 8B.

**[Table 7B]**

| components | Example 14B | Example 15B | Example 16B | Example 17B | Example 18B | Example 19B | Example 20B |
|---|---|---|---|---|---|---|---|
| ferric citrate | 597.4 | 597.4 | 597.4 | 597.4 | 597.4 | 597.4 | 597.4 |
| ferric citrate (anhydride equivalent) | (500.0) | (500.0) | (500.0) | (500.0) | (500.0) | (500.0) | (500.0) |
| polyvinyl alcohol-polyethylene glycol graft copolymer | 24.0 | 24.0 | 24.0 | 24.0 | 24.0 | 24.0 | 24.0 |
| polyvinyl alcohol-acrylic acid-methyl methacrylate copolymer | 6.0 | 6.0 | 6.0 | 6.0 | 6.0 | 6.0 | 6.0 |
| low-substituted hydroxypropylcellulose | 60.0 | --- | 42.0 | 30.0 | --- | --- | --- |
| carboxymethylcellulose | --- | 60.0 | --- | --- | --- | --- | --- |
| crystalline cellulose | --- | --- | 18.0 | 30.0 | --- | --- | --- |
| crospovidone | --- | --- | --- | --- | 60.0 | --- | --- |
| partially pregelatinized starch | --- | --- | --- | --- | --- | 60.0 | --- |
| pregelatinized starch | --- | --- | --- | --- | --- | --- | 60.0 |
| calcium stearate | 13.5 | 13.5 | 13.5 | 13.5 | 13.5 | 13.5 | 13.5 |
| total tablet (mass containing water content of ferric citrate) | 700.9 | 700.9 | 700.9 | 700.9 | 700.9 | 700.9 | 700.9 |
| total tablet (mass without water content of ferric citrate) | 603.5 | 603.5 | 603.5 | 603.5 | 603.5 | 603.5 | 603.5 |

| | | | | | | | |
|---|---|---|---|---|---|---|---|
| The numerical values in the Table show ingredients (mg) per tablet. | | | | | | | |

**[Table 8B]**

| | Example 14B | Example 15B | Example 16B | Example 17B | Example 18B | Example 19B | Example 20B |
|---|---|---|---|---|---|---|---|
| ferric citrate (anhydride equivalent) content (%) of the plain tablet (*1) | 82.9 | 82.9 | 82.9 | 82.9 | 82.9 | 82.9 | 82.9 |
| mass of polyvinyl alcohol-polyethylene glycol graft copolymer relative to 100 parts by mass of ferric citrate (anhydride equivalent) (*2) | 4.8 | 4.8 | 4.8 | 4.8 | 4.8 | 4.8 | 4.8 |
| mass of polyvinyl alcohol-acrylic acid-methyl methacrylate copolymer relative to 100 parts by mass of ferric citrate (anhydride equivalent) (*3) | 1.2 | 1.2 | 1.2 | 1.2 | 1.2 | 1.2 | 1.2 |
| mass of low-substituted hydroxypropylcellulose, carboxymethylcellulose, crystalline cellulose, crospovidone, partially pregelatinized starch or pregelatinized starch relative to 100 parts by mass of ferric citrate (anhydride equivalent) (*4) | 12.0 | 12.0 | 12.0 | 12.0 | 12.0 | 12.0 | 12.0 |

| | | | | | | | |
|---|---|---|---|---|---|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride equivalent)]x100 *4: [low-substituted hydroxypropylcellulose, carboxymethylcellulose, crystalline cellulose, crospovidone, partially pregelatinized starch or pregelatinized starch/ferric citrate (anhydride equivalent)]x100 | | | | | | | |

### Experimental Example 4B (moldability test)

The tablets obtained in Examples 14B - 20B were observed under a stereomicroscope (SZH, manufactured by OLYMPUS) at magnification x15 to confirm the presence or absence of cracks. In addition, tablet hardness was measured by a tablet hardness meter (6D, manufactured by Schleuniger).

### Experimental Example 5B (disintegration test)

The tablets obtained in Examples 14B - 20B were evaluated for the disintegration property by the same method as in Experimental Example 3B.
The results of Experimental Examples 4B and 5B are shown in Table 9B.

**[Table 9B]**

| | Example 14B | Example 15B | Example 16B | Example 17B | Example 18B | Example 19B | Example 20B |
|---|---|---|---|---|---|---|---|
| tablet hardness (N) | 201 | 206 | 217 | 222 | 172 | 157 | 142 |
| rate of observed cracks (per 10 tablets) | 0/10 | 0/10 | 2/10 | 2/10 | 5/10 | 9/10 | 10/10 |
| disintegration time (min) | 2.6 | 0.9 | 2.0 | 3.0 | 0.6 | 11.8 | 11.8 |

### Example 21B

A sieved powder (13.8028 g) obtained in Example 13B and low-substituted hydroxypropylcellulose (L-HPC LH-11, manufactured by Shin-Etsu Chemical Co., Ltd., 1.32 g) were mixed in a 50 mL container for 10 min by using a turbular mixer. Calcium stearate (the Japanese Pharmacopoeia, plant-derived, manufactured by Taihei Chemical Industrial Co., Ltd., 0.297 g) was added and the mixture was admixed by using a turbular mixer for 3 min to give a powder for tableting. The obtained powder for tableting (700.9 mg) was molded by a 50kN screw drive universal testing machine (SC-50HJ, manufactured by Tokyo Testing Machine) at compression rate 20 mm/min, compression pressure 10 kN to give a plain tablet having major axis 17.5 mm, minor axis 8 mm (capsule-shaped tablet, containing 24 mg of a polyvinyl alcohol-polyethylene glycol graft copolymer, 6 mg of polyvinyl alcohol-acrylic acid-methyl methacrylate copolymer, 60 mg of low-substituted hydroxypropylcellulose and 13.5 mg of calcium stearate).

### Example 22B

The same operation as that shown in Example 21B except that magnesium stearate (the Japanese Pharmacopoeia, plant-derived, manufactured by Taihei Chemical Industrial Co., Ltd.) was used instead of calcium stearate was performed to give a plain tablet containing ferric citrate (capsule-shaped tablet, containing a polyvinyl alcohol-polyethylene glycol graft copolymer 24 mg, a polyvinyl alcohol-acrylic acid-methyl methacrylate copolymer 6 mg, low-substituted hydroxypropylcellulose 60 mg and magnesium stearate 13.5 mg).

### Example 23B

The same operation as that shown in Example 21B except that sodium stearyl fumarate (PRUV, manufactured by JRS PHARMA) was used instead of calcium stearate was performed to give a plain tablet containing ferric citrate (capsule-shaped tablet, containing a polyvinyl alcohol-polyethylene glycol graft copolymer 24 mg, a polyvinyl alcohol-acrylic acid-methyl methacrylate copolymer 6 mg, low-substituted hydroxypropylcellulose 60 mg and sodium stearyl fumarate 13.5 mg).

### Example 24B

The same operation as that shown in Example 21B except that stearic acid (NAA-180P-1, manufactured by NOF Corporation) was used instead of calcium stearate was performed. As a result, a powder attachment (binding) was observed on the die wall surface after molding.

### Example 25B

The same operation as that shown in Example 21B except that talc (Hi-Filler #17, manufactured by Matsumura Sangyo Co. Ltd.) was used instead of calcium stearate was performed. As a result, a powder attachment (binding) was observed on the die wall surface after molding.
The component compositions of the tablets of Examples 21B - 25B are shown in Table 10B. In addition, ferric citrate (anhydride equivalent) content (%) of the plain tablet, the mass of a polyvinyl alcohol-polyethylene glycol graft copolymer relative to 100 parts by mass of ferric citrate (anhydride equivalent), the mass of a polyvinyl alcohol-acrylic acid-methyl methacrylate copolymer relative to 100 parts by mass of ferric citrate (anhydride equivalent) and the mass of low-substituted hydroxypropylcellulose relative to 100 parts by mass of ferric citrate (anhydride equivalent) are shown in Table 11B.

**[Table 10B]**

| components | Example 21B | Example 22B | Example 23B | Example 24B | Example 25B |
|---|---|---|---|---|---|
| ferric citrate | 597.4 | 597.4 | 597.4 | 597.4 | 597.4 |
| ferric citrate (anhydride equivalent) | (500.0) | (500.0) | (500.0) | (500.0) | (500.0) |
| polyvinyl alcohol-polyethylene glycol graft copolymer | 24.0 | 24.0 | 24.0 | 24.0 | 24.0 |
| polyvinyl alcohol-acrylic acid-methyl methacrylate copolymer | 6.0 | 6.0 | 6.0 | 6.0 | 6.0 |
| low-substituted hydroxypropylcellulose | 60.0 | 60.0 | 60.0 | 60.0 | 60.0 |
| calcium stearate | 13.5 | --- | --- | --- | --- |
| magnesium stearate | --- | 13.5 | --- | --- | --- |
| sodium stearyl fumarate | --- | --- | 13.5 | --- | --- |
| stearic acid | --- | --- | --- | 13.5 | --- |
| talc | --- | --- | --- | --- | 13.5 |
| total tablet (mass containing water content of ferric citrate) | 700.9 | 700.9 | 700.9 | 700.9 | 700.9 |
| total tablet (mass without water content of ferric citrate) | 603.5 | 603.5 | 603.5 | 603.5 | 603.5 |

| | | | | | |
|---|---|---|---|---|---|
| The numerical values in the Table show ingredients (mg) per tablet. | | | | | |

**[Table 11B]**

| | Example 21B | Example 22B | Example 23B | Example 24B | Example 25B |
|---|---|---|---|---|---|
| ferric citrate (anhydride equivalent) content (%) of the plain tablet(*1) | 82.9 | 82.9 | 82.9 | 82.9 | 82.9 |
| mass of polyvinyl alcohol-polyethylene glycol graft copolymer relative to 100 parts by mass of ferric citrate (anhydride equivalent) (*2) | 4.8 | 4.8 | 4.8 | 4.8 | 4.8 |
| mass of polyvinyl alcohol-acrylic acid-methyl methacrylate copolymer relative to 100 parts by mass of ferric citrate (anhydride equivalent) (*3) | 1.2 | 1.2 | 1.2 | 1.2 | 1.2 |
| mass of low-substituted hydroxypropylcellulose relative to 100 parts by mass of ferric citrate (anhydride equivalent) (*4) | 12.0 | 12.0 | 12.0 | 12.0 | 12.0 |

| | | | | | |
|---|---|---|---|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride eqvivalent)]x100 *4: [low-substituted hydroxypropylcellulose/ferric citrate (anhydride equivalent)]x100 | | | | | |

### Experimental Example 6B (tablet extrusion pressure)

When the tablets compression molded in Examples 21B - 23B were extruded from the die, a 50kN screw drive universal testing machine (SC-50HJ, manufactured by Tokyo Testing Machine) was used to extrude the tablets from the die at 20 mm/min by using the punch used for molding, and the extrusion pressure was measured.

### Experimental Example 7B (moldability test)

The tablets obtained in Examples 21B - 23B were observed under a stereomicroscope (SZH, manufactured by OLYMPUS) at magnification x15 to confirm the presence or absence of cracks. In addition, tablet hardness was measured by a tablet hardness meter (6D, manufactured by Schleuniger).

### Experimental Example 8B (disintegration test)

The tablets obtained in Examples 21B - 23B were evaluated for the disintegration property by the same method as in Experimental Example 3B.
The results of Experimental Examples 6B, 7B and 8B are shown in Table 12B.

**[Table 12B]**

| | Example 21B | Example 22B | Example 23B |
|---|---|---|---|
| tablet extrusion pressure (N) | 2.2 | 0 | 30.9 |
| tablet hardness (N) | 195 | 204 | 218 |
| rate of crack observed (per 10 tablets) | 0/10 | 0/10 | 3/10 |
| disintegration time (min) | 2.2 | 2.6 | 1.7 |

### Example 26B

A polyvinyl alcohol-polyethylene glycol graft copolymer (Kollicoat IR, manufactured by BASF, 84 g) and a polyvinyl alcohol-acrylic acid-methyl methacrylate copolymer (POVACOAT Type: F, manufactured by Daido Chemical Corporation, 21 g) were added to purified water (1295 g), and dissolved by using a propeller mixer to prepare a binding solution.
Ferric citrate (398.2 g, equivalent to anhydride 325 g) and crystalline cellulose (CEOLUS PH-102, manufactured by Asahikasei Chemicals, 56.8 g) were placed in a tumbling fluid bed dryer granulator (MP-01, manufactured by POWREX) and granulated while spraying the above-mentioned binding solution (260 g) and dried. The obtained dried granules were sieved with a stainless sieve (aperture 500 µm) to give a sieved powder.

### Example 27B

A sieved powder (211.7 g) obtained in Example 26B and low-substituted hydroxypropylcellulose (L-HPC LH-11, manufactured by Shin-Etsu Chemical Co., Ltd., 17.4 g) were mixed in a 2 L container for 10 min by using a turbular mixer. Calcium stearate (the Japanese Pharmacopoeia, plant-derived, manufactured by Taihei Chemical Industrial Co., Ltd., 3.94 g) was added and the mixture was admixed by using a turbular mixer for 3 min to give a powder for tableting. The powder for tableting was tableted by a rotary tableting machine (Correct 12HUK, manufactured by Kikusui Seisakusho Ltd.) at tableting pressure 900 kgf/punch to give capsule-shaped tablets each having major axis 14.8 mm, minor axis 6.8 mm, mass 401.8 mg (containing 12 mg of a polyvinyl alcohol-polyethylene glycol graft copolymer, 3 mg of a polyvinyl alcohol-acrylic acid-methyl methacrylate copolymer and 30 mg of low-substituted hydroxypropylcellulose per tablet).

### Example 28B

A sieved powder (211.7 g) obtained in Example 26B and low-substituted hydroxypropylcellulose (L-HPC LH-11, manufactured by Shin-Etsu Chemical Co., Ltd., 17.4 g) and crospovidone (Kollidon CL-F, manufactured by BASF, 2.9 g) were mixed in a 2 L container for 10 min by using a turbular mixer. Calcium stearate (the Japanese Pharmacopoeia, plant-derived, manufactured by Taihei Chemical Industrial Co., Ltd., 3.94 g) was added and the mixture was admixed by using a turbular mixer for 3 min to give a powder for tableting. The powder for tableting was tableted by a rotary tableting machine (Correct 12HUK, manufactured by Kikusui Seisakusho Ltd.) at tableting pressure 900 kgf/punch to give capsule-shaped tablets each having major axis 14.8 mm, minor axis 6.8 mm, mass 406.8 mg (containing 12 mg of a polyvinyl alcohol-polyethylene glycol graft copolymer, 3 mg of a polyvinyl alcohol-acrylic acid-methyl methacrylate copolymer, 30 mg of low-substituted hydroxypropylcellulose and 5 mg of crospovidone per tablet).

The component compositions of the tablets of Examples 27B and 28B are shown in Table 13B. In addition, ferric citrate (anhydride equivalent) content (%) of the plain tablet, the mass of a polyvinyl alcohol-polyethylene glycol graft copolymer relative to 100 parts by mass of ferric citrate (anhydride equivalent), the mass of a polyvinyl alcohol-acrylic acid-methyl methacrylate copolymer relative to 100 parts by mass of ferric citrate (anhydride equivalent), the mass of low-substituted hydroxypropylcellulose relative to 100 parts by mass of ferric citrate (anhydride equivalent) and the mass of crospovidone relative to 100 parts by mass of ferric citrate (anhydride equivalent) are shown in Table 14B.

**[Table 13B]**

| components | Example 27B | Example 28B |
|---|---|---|
| ferric citrate | 306.3 | 306.3 |
| ferric citrate (anhydride equivalent) | (250.0) | (250.0) |
| crystalline cellulose | 43.7 | 43.7 |
| polyvinyl alcohol-polyethylene glycol graft copolymer | 12.0 | 12.0 |
| polyvinyl alcohol-acrylic acid-methyl methacrylate copolymer | 3.0 | 3.0 |
| low-substituted hydroxypropylcellulose | 30.0 | 30.0 |
| crospovidone | --- | 5.0 |
| calcium stearate | 6.8 | 6.8 |
| total tablet (mass containing water content of ferric citrate) | 401.8 | 406.8 |
| total tablet (mass without water content of ferric citrate) | 345.5 | 350.5 |

| | | |
|---|---|---|
| The numerical values in the Table show ingredients (mg) per tablet. | | |

**[Table 14B]**

| | Example 27B | Example 28B |
|---|---|---|
| ferric citrate (anhydride equivalent) content (%) of the plain tablet(*1) | 72.4 | 71.3 |
| mass of polyvinyl alcohol-polyethylene glycol graft copolymer relative to 100 parts by mass of ferric citrate (anhydride equivalent) (*2) | 4.8 | 4.8 |
| mass of polyvinyl alcohol-acrylic acid-methyl methacrylate copolymer relative to 100 parts by mass of ferric citrate (anhydride equivalent) (*3) | 1.2 | 1.2 |
| mass of low-substituted hydroxypropylcellulose relative to 100 parts by mass of ferric citrate (anhydride equivalent) (*4) | 12.0 | 12.0 |
| mass of crospovidone relative to 100 parts by mass of ferric citrate (anhydride equivalent) (*5) | --- | 2.0 |

| | | |
|---|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride equivalent)]x100 *4: [low-substituted hydroxypropylcellulose/ferric citrate (anhydride equivalent)]x100 *5: [crospovidone/ferric citrate (anhydride equivalent)]x100 | | |

### Experimental Example 9B (moldability test)

The tablets obtained in Examples 27B and 28B were observed under a stereomicroscope (SZH, manufactured by OLYMPUS) at magnification x15 to confirm the presence or absence of cracks. In addition, tablet hardness was measured by a tablet hardness meter (6D, manufactured by Schleuniger).

### Experimental Example 10B (disintegration test)

The tablets obtained in Examples 27B and 28B were evaluated for the disintegration property by the same method as in Experimental Example 3B.

The results of Experimental Examples 9B and 10B are shown in Table 15B.

**[Table 15B]**

| | Example 27B | Example 28B |
|---|---|---|
| tablet hardness (N) | 122 | 123 |
| rate of crack observed (per 10 tablets) | 0/10 | 0/10 |
| disintegration time (min) | 1.7 | 0.8 |

The production methods of ferric citrate to be used in Example 29B are as described below.

### (Lot AW)

### Iron-containing precipitate formation step

639.5 kg (67.3 kg as Fe³⁺; 1205 mol) of aqueous ferric chloride solution was placed in a reaction container, and diluted with 1002 kg of purified water to give an aqueous ferric chloride solution containing 4.1% by mass of Fe³⁺. The aqueous ferric chloride solution was cooled to a solution temperature of 0 - 5°C. 1467.9 kg of 10% by mass of an aqueous sodium hydroxide solution cooled to a solution temperature of 0 - 5°C in advance was added dropwise to the aforementioned aqueous ferric chloride solution over 120 min while maintaining the solution temperature of 3.5 - 8.0°C to adjust the final pH to 9.22. After the completion of the dropwise addition, the obtained mixture was stirred at a temperature (solution temperature) of 3.7 - 4.7°C for 1 hr. The pH was measured to confirm that the pH of the mixture was within the range of 8.0 - 10.0.

### Washing step

The mixture obtained in the above-mentioned step was washed by filtration with 2000 L of purified water. An iron-containing crude precipitate (wet solid (1): 628.02 kg) containing filtrated ferrihydrate as a main component was washed by stirring in 1627.0 kg of purified water for 25 min. This suspension was filtered again to give an iron-containing precipitate containing ferrihydrate as a main component (wet solid (2): 530.75 kg).

### Aqueous ferric citrate solution formation step

289.30 kg (1506 mol) of citric acid was dissolved in 389.0 kg of purified water to prepare 678.3 kg of aqueous citric acid solution. 530.75 kg of the wet solid (2) obtained in the above-mentioned step and 678.3 kg of the aforementioned aqueous citric acid solution were placed in a reaction container, and slowly stirred at room temperature (about 25°C) for 69 min at about 50 rpm to form a mixture. Then, the mixture was slowly heated until the temperature (solution temperature) of the mixture reached 80°C under such conditions as to make the difference between the temperature (solution temperature) of the mixture and the external temperature fall within the range of 0 - 15°C. Thereafter, the mixture was stirred at a solution temperature of 80.0 - 81.9°C for 120 min to dissolve the iron-containing precipitate containing ferrihydrate as a main component. After confirmation of dissolution of the iron-containing precipitate containing ferrihydrate as a main component, the mixture was cooled to a solution temperature of 20 30°C. The insoluble material in the obtained mixture was removed by filtration to give an aqueous ferric citrate solution (1226.5 kg).

### Ferric citrate precipitation step

2453 kg of acetone was placed in a reaction container. 613.2 kg of the aqueous ferric citrate solution obtained in the above-mentioned step was added dropwise to acetone in the reaction container over 45 min with stirring. After the completion of the dropwise addition, the obtained mixture was stirred at a solution temperature of 24.0 - 24.6°C for 40 min. The obtained mixture was filtered to give a precipitate containing ferric citrate (wet solid (3): 425.17 kg). The obtained 425.17 kg of wet solid (3) was dried to give the object highly pure ferric citrate (yield: 154.21 kg; 91.7%).

### (Lot Ax):

Ferric citrate (lot AX) was prepared by a method similar to the production method of lot AW (yield: 154.61 kg; 91.9%).

### Example 29B

A polyvinyl alcohol-polyethylene glycol graft copolymer (Kollicoat IR, manufactured by BASF, 1.680 kg) and a polyvinyl alcohol-acrylic acid-methyl methacrylate copolymer (POVACOAT Type:F, manufactured by Daido Chemical Corporation, 0.42 kg) were added to purified water (25.9 kg) and dissolved therein by using a propeller mixer to prepare a binding solution.
As ferric citrate, a mixture of lot AW and lot AX (each 19.1624 kg) (total 38.3248 kg); equivalent to anhydride 30 kg), and crystalline cellulose (CEOLUS PH-102, manufactured by Asahikasei Chemicals, 3.4591 kg) were placed in a fluid bed dryer granulator (WSG-60, manufactured by POWREX), and the mixture was granulated by spraying the above-mentioned binding solution (24.0 kg) and dried. The obtained dried granules were sieved with a screen (aperture 1143 µm) by a screen mill (U20, manufactured by POWREX) to give a sieved powder.
To the obtained sieved powder (41.4048 kg) were added low-substituted hydroxypropylcellulose (LH-11, manufactured by Shin-Etsu Chemical Co., Ltd., 3.42 kg) and crospovidone (Kollidon CL-F, manufactured by BASF, 0.57 kg), and the mixture was admixed by a W-type blending machine (TCW-100, manufactured by Tokuju Corporation) at 29 rpm for 310 seconds. Calcium stearate (the Japanese Pharmacopoeia, calcium stearate, plant-derived, manufactured by Taihei Chemical Industrial Co., Ltd., 0.7752 kg) was added, and the mixture was admixed by a W-type blending machine (TCW-100, manufactured by Tokuju Corporation) at 29 rpm for 104 seconds to give a powder for tableting. The powder for tableting was tableted by a rotary tableting machine (Correct 12HUK, manufactured by Kikusui Seisakusho Ltd.) at tableting pressure 950 kgf/punch to give capsule-shaped plain tablets having major axis 14.8 mm, minor axis 6.8 mm, mass 405 mg.
The obtained plain tablets (12.15 kg) were coated with a coating solution obtained by mixing hypromellose (TC-5M, manufactured by Shin-Etsu Chemical Co., Ltd., 600 g), titanium oxide (Titanuim(IV) Oxide extra pure, manufactured by Merck, 200 g), talc (Hi-Filler #17, manufactured by Matsumura Sangyo Co. Ltd., 100 g), macrogol 6000 (macrogol 6000P, manufactured by NOF Corporation, 100 g), and purified water (7000 g), by an automatic coating machine (HCT-60N, manufactured by Freund Corporation) to give tablets having an about 18 mg of a coating layer per tablet (containing 12 mg of polyvinyl alcohol-polyethylene glycol graft copolymer, 3 mg of polyvinyl alcohol-acrylic acid-methyl methacrylate copolymer, 30 mg of low-substituted hydroxypropylcellulose and 5 mg of crospovidone per tablet).
The component composition of the tablet of Example 29B is shown in Table 16B. In addition, ferric citrate (anhydride equivalent) content (%) of the plain tablet, the mass of a polyvinyl alcohol-polyethylene glycol graft copolymer relative to 100 parts by mass of ferric citrate (anhydride equivalent), the mass of a polyvinyl alcohol-acrylic acid-methyl methacrylate copolymer relative to 100 parts by mass of ferric citrate (anhydride equivalent), the mass of low-substituted hydroxypropylcellulose and crystalline cellulose relative to 100 parts by mass of ferric citrate (anhydride equivalent) and the mass of crospovidone relative to 100 parts by mass of ferric citrate (anhydride equivalent) are shown in Table 17B.

**[Table 16B]**

| components | Example 29B |
|---|---|
| ferric citrate | 319.4 |
| ferric citrate (anhydride equivalent) | (250.0) |
| crystalline cellulose | 28.8 |
| polyvinyl alcohol-polyethylene glycol graft copolymer | 12.0 |
| polyvinyl alcohol-acrylic acid-methyl methacrylate copolymer | 3.0 |
| low-substituted hydroxypropylcellulose | 30.0 |
| crospovidone | 5.0 |
| calcium stearate | 6.8 |
| total tablet (mass containing water content of ferric citrate) | 405.0 |
| total tablet (mass without water content of ferric citrate) | 335.6 |
| hypromellose | 10.8 |
| titanium oxide | 3.6 |
| talc | 1.8 |
| macrogol 6000 | 1.8 |
| total coating film | 18.0 |

| | |
|---|---|
| The numerical values in the Table show ingredients (mg) per tablet. | |

**[Table 17B]**

| | Example 29B |
|---|---|
| ferric citrate (anhydride equivalent) content (%) of the plain tablet(*1) | 74.5 |
| mass of polyvinyl alcohol-polyethylene glycol graft copolymer relative to 100 parts by mass of ferric citrate (anhydride equivalent) (*2) | 4.8 |
| mass of polyvinyl alcohol-acrylic acid-methyl methacrylate copolymer relative to 100 parts by mass of ferric citrate (anhydride equivalent) (*3) | 1.2 |
| mass of low-substituted hydroxypropylcellulose and crystalline cellulose relative to 100 parts by mass of ferric citrate (anhydride equivalent) (*4) | 23.5 |
| mass of crospovidone relative to 100 parts by mass of ferric citrate (anhydride equivalent) (*5) | 2.0 |

| | |
|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride equivalent)]x100 *4: [low-substituted hydroxypropylcellulose and crystalline cellulose/ferric citrate (anhydride equivalent)]x100 *5: [crospovidone/ferric citrate (anhydride equivalent)]x100 | |

The production methods of ferric citrate to be used in Example 30B are as described below.

### (Lot AR):

### Iron containing precipitate formation step

60.5 kg (6.7 kg as Fe³⁺; 120.0 mol) of aqueous ferric chloride solution was placed in a reaction container, and diluted with 102.9 kg of purified water to give an aqueous ferric chloride solution containing 4.1% by mass of Fe³⁺. The aqueous ferric chloride solution was cooled to a solution temperature of 0 - 5°C. 139.4 kg of 10% by mass of an aqueous sodium hydroxide solution cooled to a solution temperature of 0 - 5°C in advance was added dropwise to the aforementioned aqueous ferric chloride solution over 120 min while maintaining the solution temperature of 0 - 4.2°C to adjust the final pH to 9.05. After the completion of the dropwise addition, the obtained mixture was stirred at a temperature (solution temperature) of 1.6 - 3.8°C for 1 hr. The pH was measured to confirm that the pH of the mixture was within the range of 8.0 - 10.0.

### Washing step

The mixture obtained in the above-mentioned step was washed by filtration with 120 kg of purified water. An iron-containing crude precipitate (wet solid (1) : 70.52 kg) containing filtrated ferrihydrate as a main component was washed by stirring in 162.7 kg of purified water for 55 min. This suspension was filtered again to give an iron-containing precipitate containing ferrihydrate as a main component (wet solid (2): 53.26 kg).

### Aqueous ferric citrate solution formation step

28.9 kg (150.5 mol) of citric acid was dissolved in 38.74 kg of purified water to prepare 67.64 kg of aqueous citric acid solution. 53.26 kg of the wet solid (2) obtained in the above-mentioned step and 67.64 kg of the aforementioned aqueous citric acid solution were placed in a reaction container, and slowly stirred at room temperature (about 25°C) for 70 min at about 67 rpm to form a mixture. Then, the mixture was slowly heated until the temperature (solution temperature) of the mixture reached 80°C under such conditions as to make the difference between the temperature (solution temperature) of the mixture and the external temperature fall within the range of 0 - 15°C. Thereafter, the mixture was stirred at a solution temperature of 80.1 - 84.0°C for 120 min to dissolve the iron-containing precipitate containing ferrihydrate as a main component. After confirmation of dissolution of the iron-containing precipitate containing ferrihydrate as a main component, the mixture was cooled to a solution temperature of 20 - 30°C. The insoluble material in the obtained mixture was removed by filtration to give an aqueous ferric citrate solution (118.0 kg).

### Ferric citrate precipitation step

471.8 kg of 95% by mass acetone (acetone containing 5% by mass of water) was placed in a reaction container. 118.0 kg of the aqueous ferric citrate solution obtained in the above-mentioned step was added dropwise to 95% by mass acetone in the reaction container over 25 min with stirring. After the completion of the dropwise addition, the obtained mixture was stirred at a solution temperature of 21.1 - 22.2°C for 40 min. The obtained mixture was filtered to give a precipitate containing ferric citrate (wet solid (3): 74.08 kg). The obtained 74.08 kg of wet solid (3) was dried to give the object highly pure ferric citrate (yield: 25.86 kg; 78.86%).

### (Lot BD:

Ferric citrate of lot BD was prepared by a method similar to that in lot AW except that, in the iron-containing precipitate formation step of lot AW, "an aqueous sodium hydroxide solution was added dropwise to an aqueous ferric chloride solution over 115 min while maintaining the solution temperature of 2.6 - 7.5°C to adjust the final pH to 9.09" instead of "an aqueous sodium hydroxide solution was added dropwise to the aforementioned aqueous ferric chloride solution over 120 min while maintaining the solution temperature of 3.5 - 8.0°C to adjust the final pH to 9.22" (yield: 156.09 kg; 92.3%).

### (Lot BE):

Ferric citrate of lot BE was prepared by a method similar to that in lot AW except that, in the iron-containing precipitate formation step of lot AW, "an aqueous sodium hydroxide solution was added dropwise to an aqueous ferric chloride solution over 162 min while maintaining the solution temperature of 2 .4 - 8.6°C to adjust the final pH to 9.21" instead of "an aqueous sodium hydroxide solution was added dropwise to the aforementioned aqueous ferric chloride solution over 120 min while maintaining the solution temperature of 3.5 - 8.0°C to adjust the final pH to 9.22"
(yield: 150.43 kg; 92.1%).

### (Lot BF) :

Ferric citrate of lot BF was prepared by a method similar to that in lot AW except that, in the iron-containing precipitate formation step of lot AW, "an aqueous sodium hydroxide solution was added dropwise to an aqueous ferric chloride solution over 162 min while maintaining the solution temperature of 2.4 - 8.6°C to adjust the final pH to 9.21" instead of "an aqueous sodium hydroxide solution was added dropwise to the aforementioned aqueous ferric chloride solution over 120 min while maintaining the solution temperature of 3.5 - 8.0°C to adjust the final pH to 9.22"
(yield: 152.30 kg; 92.8%).

### Example 30B

A polyvinyl alcohol-polyethylene glycol graft copolymer (Kollicoat IR, manufactured by BASF, 1.6800 kg) and a polyvinyl alcohol-acrylic acid-methyl methacrylate copolymer (POVACOAT Type:F, manufactured by Daido Chemical Corporation, 0.4201 kg) were added to purified water (25.9 kg) and dissolved therein by using a propeller mixer to prepare a binding solution.
As ferric citrate, a mixture of lot AR (9.2540 kg), lot BD (9.4352 kg), lot BE (9.1210 kg) and lot BF (9.1585 kg) (total 36.9687 kg); equivalent to anhydride 30 kg), and crystalline cellulose (CEOLUS PH-102, manufactured by Asahikasei Chemicals, 4.8808 kg) were placed in a fluid bed dryer granulator (WSG-60, manufactured by POWREX), and the mixture was granulated by spraying the above-mentioned binding solution (24.0 kg) and dried. The obtained dried granules were sieved with a screen (aperture 1143 µm) by a screen mill (U20, manufactured by POWREX) to give a sieved powder.
To the obtained sieved powder (41.4670 kg) were added low-substituted hydroxypropylcellulose (LH-11, manufactured by Shin-Etsu Chemical Co., Ltd., 3.4200 kg) and crospovidone (Kollidon CL-F, manufactured by BASF, 0.5700 kg), and the mixture was admixed by a W-type blending machine (TCW-100, manufactured by Tokuju Corporation) at 29 rpm for 310 seconds. Calcium stearate (the Japanese Pharmacopoeia, calcium stearate, plant-derived, manufactured by Taihei Chemical Industrial Co., Ltd., 0.7752 kg) was added, and the mixture was admixed by a W-type blending machine (TCW-100, manufactured by Tokuju Corporation) at 29 rpm for 104 seconds to give a powder for tableting. The powder for tableting was tableted by a rotary tableting machine (Correct 12HUK, manufactured by Kikusui Seisakusho Ltd.) at tableting pressure 1000 kgf/punch to give capsule-shaped plain tablets having major axis 14.8 mm, minor axis 6.8 mm, mass 405.6 mg.
The obtained plain tablets (324.3 g) were coated with a coating solution obtained by mixing hypromellose (TC-5M, manufactured by Shin-Etsu Chemical Co., Ltd., 60 g), titanium oxide (Titanuim(IV) Oxide extra pure, manufactured by Merck, 20 g), talc (Hi-Filler #17, manufactured by Matsumura Sangyo Co. Ltd., 10 g), macrogol 6000 (macrogol 6000P, manufactured by NOF Corporation, 10 g), and purified water (700 g), by an automatic coating machine (HC-LABO, manufactured by Freund Corporation) to give tablets having an about 18 mg of a coating layer per tablet (containing 12 mg of polyvinyl alcohol-polyethylene glycol graft copolymer, 3 mg of polyvinyl alcohol-acrylic acid-methyl methacrylate copolymer, 30 mg of low-substituted hydroxypropylcellulose and 5 mg of crospovidone per tablet).
The component composition of the tablet of Example 30B is shown in Table 18B. In addition, ferric citrate (anhydride equivalent) content (%) of the plain tablet, the mass of a polyvinyl alcohol-polyethylene glycol graft copolymer relative to 100 parts by mass of ferric citrate (anhydride equivalent), the mass of a polyvinyl alcohol-acrylic acid-methyl methacrylate copolymer relative to 100 parts by mass of ferric citrate (anhydride equivalent), the mass of low-substituted hydroxypropylcellulose, carboxymethylcellulose and crystalline cellulose relative to 100 parts by mass of ferric citrate (anhydride equivalent), and the mass of crospovidone relative to 100 parts by mass of ferric citrate (anhydride equivalent) are shown in Table 19B.

**[Table 18B]**

| components | Example 30B |
|---|---|
| ferric citrate (*1) | 308.1 |
| ferric citrate (anhydride equivalent) | 250.0 |
| solid content of ferric citrate (*2) | 250.5 |
| water content of ferric citrate | 57.6 |
| crystalline cellulose | 40.7 |
| polyvinyl alcohol-polyethylene glycol graft copolymer | 12.0 |
| polyvinyl alcohol-acrylic acid-methyl methacrylate copolymer | 3.0 |
| low-substituted hydroxypropylcellulose | 30.0 |
| crospovidone | 5.0 |
| calcium stearate | 6.8 |
| total tablet (mass containing water content of ferric citrate) | 405.6 |
| total tablet (mass without water content of ferric citrate) | 348.0 |
| hypromellose | 10.8 |
| titanium oxide | 3.6 |
| talc | 1.8 |
| macrogol 6000 | 1.8 |
| total coating film | 18.0 |

| | |
|---|---|
| The numerical values in the Table show ingredients (mg) per tablet. *1: value amended by water content of ferric citrate and quantitative value of ferric citrate *2: solid content including impurities in ferric citrate | |

**[Table 19B]**

| | Example 30B |
|---|---|
| ferric citrate (anhydride equivalent) content (%) of the plain tablet (*1) | 71.9 |
| mass of polyvinyl alcohol-polyethylene glycol graft copolymer relative to 100 parts by mass of ferric citrate (anhydride equivalent) (*2) | 4.8 |
| mass of polyvinyl alcohol-acrylic acid-methyl methacrylate copolymer relative to 100 parts by mass of ferric citrate (anhydride equivalent) (*3) | 1.2 |
| mass of low-substituted hydroxypropylcellulose, carboxymethylcellulose and crystalline cellulose relative to 100 parts by mass of ferric citrate (anhydride equivalent) (*4) | 28.3 |
| mass of crospovidone relative to 100 parts by mass of ferric citrate (anhydride equivalent) (*5) | 2.0 |

| | |
|---|---|
| *1: [ferric citrate (anhydride equivalent)/total tablet (mass without water content of ferric citrate)]x100 *2: [polyvinyl alcohol-polyethylene glycol graft copolymer/ferric citrate (anhydride equivalent)]x100 *3: [polyvinyl alcohol-acrylic acid-methyl methacrylate copolymer/ferric citrate (anhydride equivalent)]x100 *4: [low-substituted hydroxypropylcellulose, carboxymethylcellulose and crystalline cellulose/ferric citrate (anhydride equivalent)]x100 *5: [crospovidone/ferric citrate (anhydride equivalent)]x100 | |

### Experimental Example 11B (moldability test)

The tablets (plain tablets) obtained in Examples 29B and 30B were observed under a stereomicroscope (SZH, manufactured by OLYMPUS) at magnification x15 to confirm the presence or absence of cracks. In addition, tablet hardness was measured by a tablet hardness meter (6D, manufactured by Schleuniger).

### Experimental Example 12B (disintegration property test)

The tablets (plain tablets and coated tablets) obtained in Examples 29B and 30B were evaluated for the disintegration property by the same method as in Experimental Example 3B.

The results of Experimental Examples 11B and 12B are shown in Table 20B.

**[Table 20B]**

| | | Example 29B | Example 30B |
|---|---|---|---|
| tablet hardness (N) | | 142 | 128 |
| rate of crack observed (per 60 tablets or 50 tablets) | | 1/60 | 0/50 |
| disintegration time (min) | plain tablet | 0.8 | 1.1 |
| | coated tablet | 1.7 | 2.7 |

### Experimental Example 13B

The tablets obtained in Examples 29B and 30B were subjected to a dissolution test under the conditions shown in Table 21B, and the dissolution property was evaluated.

**[Table 21B]**

| | | |
|---|---|---|
| sample amount | | 1 tablet (containing ferric citrate equivalent to anhydride 250 mg per tablet) |
| method | | The Japanese Pharmacopoeia 15, General Tests Dissolution Test Method 2nd method (Paddle Method) |
| conditions | paddle rotation number | 50 rpm |
| | test solution | The Japanese Pharmacopoeia 15, Dissolution Test 2nd fluid, 900 mL |
| | ferric citrate quantification method | color is developed with 1,10-phenanthrolin and absorbance is measured. |
| dissolution test apparatus | | automatic dissolution test apparatus (NTR-6100 or NTR-6100A, manufactured by Toyama Sangyo Co. Ltd.) |

| | | |
|---|---|---|
| In the Table, The Japanese Pharmacopoeia 15 means the Japanese Pharmacopoeia, 15th Edition. | | |

The results are shown in Fig. 1B.

### Industrial Applicability

According to the present invention, a new tablet containing ferric citrate can be provided.

## Claims

1. A tablet comprising (1) ferric citrate, (2) a polyvinyl alcohol-polyethylene glycol graft copolymer and (3) a polyvinyl alcohol-acrylic acid-methyl methacrylate copolymer.

2. The tablet according to claim 1, further comprising one or more selected from the group consisting of low-substituted hydroxypropylcellulose, crystalline cellulose and carboxymethylcellulose.

3. The tablet according to claim 1 or 2, further comprising a lubricant.

4. The tablet according to any one of claims 1 to 3, further comprising crospovidone.

5. The tablet according to any one of claims 1 to 4, wherein the ferric citrate equivalent to anhydride is contained at a ratio of not less than 70 parts by mass relative to 100 parts by mass of a plain tablet free of water content of the ferric citrate.

6. The tablet according to any one of claims 1 to 5, which is coated.

7. Use of the tablet according to any one of claims 1 to 6 as a prophylactic or therapeutic agent of hyperphosphatemia.

8. A tablet comprising ferric citrate as a medicinal active ingredient and a pharmaceutically acceptable carrier, wherein the content of the medicinal active ingredient is high.

9. The tablet according to claim 8, wherein the pharmaceutically acceptable carrier is comprised of a polyvinyl alcohol-polyethylene glycol graft copolymer and a polyvinyl alcohol-acrylic acid-methyl methacrylate copolymer.

10. The tablet according to claim 8 or 9, further comprising one or more selected from the group consisting of low-substituted hydroxypropylcellulose, crystalline cellulose and carboxymethylcellulose.

11. The tablet according to any one of claims 8 to 10, further comprising a lubricant.

12. The tablet according to any one of claims 8 to 11, further comprising crospovidone.

13. The tablet according to any one of claims 8 to 12, wherein the ferric citrate equivalent to anhydride is contained at a ratio of not less than 70 parts by mass relative to 100 parts by mass of a plain tablet free of water content of the ferric citrate.

14. The tablet according to any one of claims 8 to 13, which is coated.

15. Use of the tablet according to any one of claims 8 to 14 as a prophylactic or therapeutic agent of hyperphosphatemia.
